(19) Europäisches Patentamt
European Patent Office
Office européen des brevets

(11) **EP 2 980 584 B1**

(12) **EUROPEAN PATENT SPECIFICATION**

(45) Date of publication and mention
of the grant of the patent:
**12.09.2018 Bulletin 2018/37**

(51) Int Cl.:
**F24H 4/04** *(2006.01)*    **F24D 17/02** *(2006.01)*
**F24D 19/00** *(2006.01)*    **G01N 33/18** *(2006.01)*

(21) Application number: **13879951.5**

(22) Date of filing: **18.12.2013**

(86) International application number:
**PCT/JP2013/083892**

(87) International publication number:
**WO 2014/155868 (02.10.2014 Gazette 2014/40)**

(54) **METHOD FOR CHECKING WATER QUALITY, WATER QUALITY CHECKING DEVICE, AND HOT-WATER FEEDER SYSTEM**

VERFAHREN ZUR PRÜFUNG DER WASSERQUALITÄT, VORRICHTUNG ZUR PRÜFUNG DER WASSERQUALITÄT UND HEISSWASSERZUFUHRSYSTEM

PROCÉDÉ DE VÉRIFICATION DE LA QUALITÉ DE L'EAU, DISPOSITIF DE VÉRIFICATION DE LA QUALITÉ DE L'EAU ET SYSTÈME D'ALIMENTATION D'EAU CHAUDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2013 JP 2013071777**

(43) Date of publication of application:
**03.02.2016 Bulletin 2016/05**

(73) Proprietor: **Mitsubishi Electric Corporation Tokyo 100-8310 (JP)**

(72) Inventors:
- **NODA Seiji**
  **Tokyo 100-8310 (JP)**
- **MIYA Kazuhiro**
  **Tokyo 100-8310 (JP)**
- **FURUKAWA Seiji**
  **Tokyo 100-8310 (JP)**
- **SUZUKI Kazutaka**
  **Tokyo 100-8310 (JP)**
- **AOYAGI Yoshiro**
  **Tokyo 100-8310 (JP)**
- **INABA Yoshitsugu**
  **Tokyo 100-8310 (JP)**
- **IIJIMA Shigeru**
  **Tokyo 100-8310 (JP)**

(74) Representative: **Sajda, Wolf E. et al Meissner Bolte Patentanwälte Rechtsanwälte Partnerschaft mbB Widenmayerstraße 47 80538 München (DE)**

(56) References cited:
**WO-A1-2011/045878    JP-A- H08 224 595
JP-A- H09 174 092    JP-A- 2002 066 576
JP-A- 2002 066 576**

- **Daniel P.E. Robinette: "A New Dynamic Method for Quantifying the Precipitation of Mineral Species from Aqueous Solutions within Industrial Process Equipment", , 5 July 2011 (2011-07-05), XP055304742, Retrieved from the Internet: URL:http://www.merrick.com/merrickandcompany/media/resources/energy/whitepapers/dynamic-water-re-use-strategy.pdf?ext=.pdf [retrieved on 2016-09-22]**
- **H. M. MÜLLER-STEINHAGEN ET AL: "Comparison of indices for the scaling and corrosion tendency of water", CANADIAN JOURNAL OF CHEMICAL ENGINEERING, vol. 66, no. 6, 1 December 1988 (1988-12-01), pages 1005-1007, XP055279585, US, CA ISSN: 0008-4034, DOI: 10.1002/cjce.5450660617**

- HAWTHORN ET AL: "Heat transfer: Solving scaling problems at the design stage", CHEMICAL ENGINEERING RESEARCH AND DESIGN, PART A, INSTITUTION OF CHEMICAL ENGINEERS, XX, vol. 87, no. 2, 1 February 2009 (2009-02-01), pages 193-199, XP025897286, ISSN: 0263-8762, DOI: 10.1016/J.CHERD.2008.08.011 [retrieved on 2008-10-18]
- JITKA MACADAM ET AL: "Calcium carbonate scale formation and control", RE/VIEWS IN ENVIRONMENTAL SCIENCE & BIO/TECHNOLOGY, KLUWER ACADEMIC PUBLISHERS, DO, vol. 3, no. 2, 1 June 2004 (2004-06-01), pages 159-169, XP019265768, ISSN: 1572-9826
- ZARGA Y ET AL: "Study of calcium carbonate and sulfate co-precipitation", CHEMICAL ENGINEERING SCIENCE, OXFORD, GB, vol. 96, 4 April 2013 (2013-04-04), pages 33-41, XP028553503, ISSN: 0009-2509, DOI: 10.1016/J.CES.2013.03.028
- H. M. MULLER-STEINHAGEN ET AL.: 'Comparison of Indices for the Scaling and Corrosion Tendency of Water' THE CANADIAN JOURNAL OF CHEMICAL ENGINEERING vol. 66, no. ISSUE, December 1988, pages 1005 - 1007, XP055279585

**Description**

Technical Field

The present invention relates to, in a heat pump heat-exchanging type hot-water feeder and the like, a method for checking water quality, a water quality checking device, and a hot-water feeder system with a water quality that builds up calcium scales on a heat exchanger.

Background Art

Hot-water feeders that supply hot water to bathrooms or kitchens are broadly classified into an electric hot-water feeder, a gas hot-water feeder, an oil hot-water feeder, or the like. All of these hot-water feeders include a heat exchanger to transfer heat to water. Recently, heat pump heat-exchanger type electric hot-water feeders (heat pump hot-water feeders) are getting particular attention in view of energy saving and reducing carbon dioxide as a countermeasure for global warming.

Principles of a heat pump hot-water feeder are to transfer atmospheric heat to a heating medium, and to boil the water by this heat. That is, more specifically, the repetition (a cold cycle) of transferring, to water through the heat exchanger, heat that is generated when a gas is compressed, and bringing back the temperature of the heating medium to the atmospheric temperature again by the cold air produced when the gas is expanded.

In theory, it is not possible to obtain thermal energy that is equal to or greater than input energy. However, the heat pump hot-water feeder employs a mechanism that utilizes atmospheric heat, and thus a greater amount of thermal energy than the energy necessary for operation is available.

In order to transfer heat to water, it is very important for a heat exchanger in heat pump hot-water feeders to always maintain a clean heat transfer surface. Having an unclean wall surface of the heat transfer surface reduces the effective heat transfer area, and also deteriorates the performance of heat transfer. Further accumulation of unclean substances increases the pressure loss generated when water flows through the heat exchanger, and in the worst case, causing the flow passage to clog.

In particular, in a section of the water where high hardness components (calcium ions) are contained, inorganic compound salt with low solubility, that is so-called scales (calcium scales), mainly containing calcium carbonate are deposited by heating, and are built up in the heat exchanger. The problem of calcium scale buildup is not unique to heat pump hot-water feeders, but such a problem also occurs in electric hot-water feeders, gas hot-water feeders, oil hot-water feeders, or the like. This is attributed to the water quality that invariably exists whenever the water is heated to a predetermined temperature.

Calcium carbonate ($CaCO_3$) is produced by a reaction of calcium ion ($Ca^{2+}$) with carbonate ion ($CO_3^{2-}$) in water. Patent Literature 1 and Patent Literature 2 disclose, as a method for checking the water quality that builds up calcium scales, a method of determining the water quality based on the calcium hardness corresponding to the amount of calcium, an alkali level corresponding to the amount of carbonate ions, a pH, and a water temperature, and by performing a control so as to suppress scale build-up.

Calcium ion also reacts with phosphate ion, and produces calcium phosphate ($Ca_4(PO_4)_3$) with low solubility. Patent Literature 3 discloses that calcium phosphate is deposited in a supersaturation condition when a relationship among a pH/temperature factor, a calcium factor, and a phosphate factor that are calculated based on a pH, a calcium ion concentration, and a phosphate ion concentration in actual drain water satisfies pH/temperature factor > calcium factor + phosphate factor. Patent Literature 3 also discloses a method for suppressing the formation of scales when processing calcium-containing drain water that utilizes the production of calcium phosphate.

List of Citations

Patent Literature

Patent Literature 1: Unexamined Japanese Patent Application Kokai Publication JP S61-220 793 A
Patent Literature 2: International Publication No. WO 2011/045878
Patent Literature 3: Unexamined Japanese Patent Application Kokai Publication JP 2002-086 190 A.

## Summary of the Invention

### Technical Problem

According to the method disclosed in Patent Literature 1 and Patent Literature 2, however, the water quality is determined based on only the calcium hardness corresponding to the amount of calcium, the alkali level corresponding to the amount of carbonate ions, the pH, and the water temperature as described above. Patent Literature 3 merely discloses, with respect to the condition relevant to the supersaturation of calcium phosphate, corresponding deposition characteristics of calcium phosphate ($Ca_4(PO_4)_3$) that is produced by a reaction of calcium ion with phosphate.

That is, according to the conventional water quality checking for calcium scale build-up, in general, the water quality check is carried out based only on the calcium ion concentration, the carbon ion concentration, the hydrogen ion concentration (pH), and the water temperature, and is the calcium scale build-up due to the effects of other factors in the water quality is not taken into consideration.

The present invention has been made in view of the aforementioned circumstances, and an object of the present invention is to more precisely and accurately check the water quality that causes build-up of calcium scales.

### Solution to the Problem

To accomplish the above object, the inventors of the present invention keenly studied and found that a deposition rate of calcium carbonate remarkably changes in accordance with a concentration of dissolved ion (concentration of ion that is possibly contained in tap water, such as an orthophosphoric ion concentration ($HPO_4^{2-}$ concentration) that is monomolecular phosphoric acid, a magnesium ion concentration ($Mg^{2+}$ concentration), or a silicate ion concentration) that reacts with calcium ion and that produces low-solubility salt although the calcium ion concentration, the carbonate ion concentration, the hydrogen ion concentration (pH), and the water temperature in a solution are in equal measures (see Examples).

Hence, a method for checking a water quality according to the present invention is the method according to claims 1-6.

### Advantageous Effects of Invention

According to the present invention, an effect of a dissolved ion that reacts with calcium ion and that produces salt with low solubility to a deposition rate of calcium carbonate is taken into consideration, and thus the water quality that builds up calcium scales can be more precisely and accurately checked.

## Brief Description of Drawings

FIG. 1    is a block diagram illustrating a water quality checking device according to the present invention;

FIG. 2    is a flowchart illustrating a method for checking a water quality according to a first embodiment of the present invention;

FIG. 3    is a flowchart illustrating a method for checking a water quality according to a second embodiment of the present invention;

FIG. 4    is a flowchart illustrating a method for checking a water quality according to a third embodiment of the present invention;

FIG. 5    is a schematic diagram illustrating a structure of a coolant circuit and a water circuit in a heat pump heat-exchanger type hot-water feeder system;

FIG. 6    is a schematic diagram illustrating a structure of a hot-water feeder system according to a fourth embodiment of the present invention;

FIG. 7    is a schematic diagram illustrating a structure of a hot-water feeder system according to a fifth embodiment of the present invention;

FIG. 8    is a schematic diagram illustrating a structure of a hot-water feeder system according to a sixth embodiment of the present invention;

FIG. 9    is a schematic diagram illustrating a structure of a hot-water feeder system according to a seventh embodiment of the present invention;

FIG. 10   is a schematic diagram illustrating a structure of a hot-water feeder system according to a modified example of the seventh embodiment;

FIG. 11   is a diagram illustrating a curled fiber in a structure of a scale filtrating body;

FIG. 12    is a diagram illustrating a structure of a scale depositing test device applied in an example;

FIG. 13    is a diagram illustrating a water quality analysis result before heating according to a first example;

FIG. 14    is a diagram illustrating a change in pH, Ca hardness, and M-alkalinity after heating according to the first example;

FIG. 15    is a diagram illustrating a time change in electrical conductivity of sample water during heating according to the first example;

FIG. 16    is a diagram illustrating a value of a modified supersaturation coefficient to an orthophosphoric acid concentration according to the first example;

FIG. 17    is a diagram illustrating a water quality analysis result before heating according to a second example; and

FIG. 18    is a diagram illustrating a time change in electrical conductivity of sample water during heating according to the second example.

Description of Embodiments

As described above, the inventors of the present invention have found that the deposition rate of calcium carbonate remarkably changes due to the effects of the concentration of a dissolved ion that reacts with calcium ions and that produces salt with low solubility although the calcium ion concentration, the carbonate ion concentration, the hydrogen ion concentration (pH), and the water temperature in a solution are in equal measure (see Examples). Hence, first of all, a water quality checking device and a method for checking a water quality that have the effect of the concentration of a dissolved ion that reacts with calcium ion to produce salt with low solubility taken into consideration is described in greater detail including theoretical explanations.

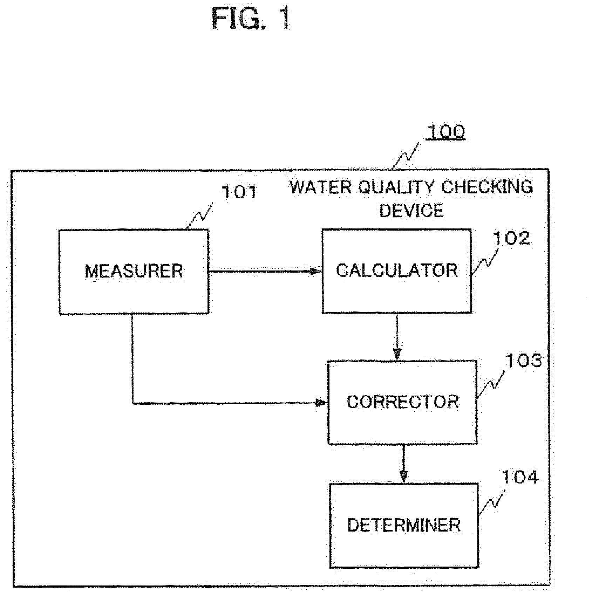

FIG, 1 is a block diagram illustrating a water quality checking device according to the present invention. As illustrated in FIG. 1, a water quality checking device 100 includes a measurer 101, a calculator 102, a corrector 103, and a determiner 104. The measurer 101 measures respective values of a calcium ion concentration, a carbonate ion concentration, a hydrogen ion concentration, a water temperature, and a concentration of a dissolved ion (orthophosphoric ion, magnesium ion, or the like) that reacts with calcium ion and that produces salt with low solubility in a solution subjected to water quality checking.

The calculator 102 calculates a supersaturation coefficient of calcium carbonate in the solution based on the measured calcium ion concentration, carbonate ion concentration, hydrogen ion concentration, and water temperature. The corrector 103 applies the concentration value of the dissolved ion that reacts with calcium ion to produce salt with low solubility, and calculates a modified supersaturation coefficient that is corrected with the effects of the calcium carbonate to the deposition rate being taken into consideration.

The determiner 104 determines the water quality based on the modified supersaturation coefficient that has been calculated. The expression "determining a water quality", in the present application is intended to mean, for example, to determine whether or not a countermeasure against scales is necessary in consequence, or to check a time (time period) at which cleaning or replacing of a heat exchanger becomes necessary, or to determine the necessity of cleaning or replacement. As discussed later in a second embodiment and a third embodiment, the determiner 104 may include, in some cases, calculation means that calculates a desired numeric value in each embodiment using accumulated specific data and based on the numeric values calculated by the corrector 103. Specific schemes of determination means are described later in more detail in the first embodiment to the third embodiment.

The inventors of the present invention studied the effects of phosphate ion to deposition characteristics of calcium carbonate while applying the homogeneous nucleation theory (see, for example, Yoshihiko Gotou, "Crystal Growth", pp. 29-44) that relates to solid nucleation. When a supersaturation coefficient of calcium carbonate is $S_1$, a water temperature is T (K), interfacial energy between a deposition nucleus and water is $\gamma$ ($J/m^2$), the Boltzmann constant is k (J/K), and a collision frequency of ion molecules in a solution is A (1/s), a deposition rate R of calcium carbonate can be given by the following formula.

[Formula 1]

$$R = A \exp\left[ -\frac{16\pi\gamma^3}{3k^3 T^3 \left(\ln S_1\right)^2} \right]$$

In addition, when a calcium concentration in the solution is $[Ca^{2+}]$, a carbonate ion concentration is $[CO_3{}^{2-}]$, an activity coefficient of divalent ion is $f_D$, and a solubility product of calcium carbonate is $K_{sp}$, the supersaturation coefficient

5

$S_1$ of calcium carbonate can be given by the following formula. The calculator 102 illustrated in FIG. 1 calculates the supersaturation coefficient $S_1$ of calcium carbonate by applying each value measured by the measurer 101 through the following formula.

[Formula 2]

$$S_1 = [Ca^{2+}] [CO_3^{2-}] f_D^2 / K_{sp}$$

The solubility product $K_{sp}$ is a physical property (see, for example, Plummer et al., Geochimica Et Cosmochimica Acta, 1982. 46(6): pp. 1011-1040) that is defined by a temperature. When, for example, the water temperature is 10 °C, 20 °C, or 60 °C, the solubility product is $3.89 \times 10^{-9}$, $3.53 \times 10^{-9}$, or $1.74 \times 10^{-9}$, respectively. That is, the higher the temperature is, the smaller the solubility product becomes, and the supersaturation coefficient $S_1$ for a certain solution composition becomes greater. This indicates that the calcium carbonate is likely to deposit at a high temperature. Conversely, the activity coefficient $f_D$ of the divalent ion varies depending on the electrical conductivity of the aqueous solution and the hydrogen ion concentration, but as for ordinary tap water, the activity coefficient is within a range between 0.6 and 0.8.

When, for example, the orthophosphoric ion is the dissolved ion that reacts with calcium ion and that produces salt with low solubility, in general, the water temperature and the supersaturation coefficient $S_1$ should normally be constant when the calcium concentration, the carbonate ion concentration, the hydrogen ion concentration (pH) in the solution, and the water temperature are in equal measure. Since Boltzmann constant k and a collision frequency A remain unchanged, the effect of the orthophosphoric ion concentration in the solution to the deposition rate of calcium carbonate can be explained as being caused by a change in the interfacial energy $\gamma$ between the deposition nucleus and the water from $\gamma \to \gamma'$ (see the following formula).

[Formula 3]

$$R = A \exp\left[ - \frac{16\pi(\gamma')^3}{3k^3 T^3 (\ln S_1)^2} \right]$$

Next, a modified supersaturation coefficient $S_2$ that satisfies the following formula is introduced in order to correct the effects of the orthophosphoric ion addition to the deposition rate R of the calcium carbonate.

[Formula 4]

$$R = A \exp\left[ - \frac{16\pi\gamma^3}{3k^3 T^3 (\ln S_2)^2} \right]$$

With the orthophosphoric ion concentration being as $C_P$, the Formula 4 above was applied, and a measurement result of a deposition rate R of the calcium carbonate in a first example to be discussed later, and that of the orthophosphoric ion concentration Cp were analyzed. As a result, it becomes clear that when $C_P$ is smaller than 3.0 mg/L, a relational expression within the following range is established between the modified supersaturation coefficient $S_2$ and the supersaturation coefficient $S_1$. That is, the modified supersaturation coefficient $S_2$ falls in the range between 0.6 and 1.4 times as much as the supersaturation coefficient $S_1$ normalized by a denominator that is a polynomial of the orthophosphoric ion concentration $C_P$. The median value in this case is 1.0 time. A representative value is calculated using the median value.

[Formula 5]

$$\frac{0.6S_1}{(-0.29C_p'^2 + 2.0C_p + 1)} \le S_2 \le \frac{1.4S_1}{(-0.29C_p'^2 + 2.0C_p + 1)}$$

Since the deposition rate R of the calcium carbonate is constant when $C_P$ is equal to or greater than 3.0 mg/L, in this case, it becomes clear that a relational expression within the following range is established between the modified supersaturation coefficient $S_2$ and the supersaturation coefficient $S_1$. In this case, the median value is $0.227S_1$. The representative value is calculated using the median value. Accordingly, the corrector 103 as illustrated in FIG. 1 applies the above formula 5 or the following formula 6 based on the value of the orthophosphoric ion concentration measured by the measurer 101, and calculates, from the supersaturation coefficient $S_1$, the modified supersaturation coefficient $S_2$ that is corrected taking into consideration the effect of the orthophosphoric ion on the deposition rate of calcium carbonate.

[Formula 6]

$$0.136S_1 \le S_2 \le 0.318S_1$$

According to the water quality checking that takes into consideration the effect of the orthophosphoric ion concentration, first of all, the calcium ion concentration, the carbonate ion concentration, the hydrogen ion concentration, and the water temperature are measured, and the orthophosphoric ion concentration is further measured. Subsequently, the supersaturation coefficient $S_1$ of the calcium carbonate out of consideration for any dissolved ions other than the calcium ion concentration, the carbonate ion concentration, and the hydrogen ion concentration is calculated. Next, a correction and a calculation for the modified supersaturation coefficient $S_2$ are carried out based on the orthophosphoric ion concentration. The determiner 104 illustrated in FIG. 1 determines the water quality by applying this modified supersaturation coefficient $S_2$.

Conversely, when, for example, the magnesium ion is the dissolved ion that reacts with the calcium ion and that produces salt with low solubility, it becomes clear that a certain relational expression can also be established. With the magnesium ion concentration being as $C_M$ (mol/L), and the calcium ion concentration being as Cc (mol/L), the aforementioned formula 4 was applied to analyze the measurement result of the deposition rate R of calcium carbonate in a second example to be discussed later, and that of the magnesium ion concentration $C_M$. In this case, it is found that a relational expression within the following range is established between the modified supersaturation coefficient $S_2$ and the supersaturation coefficient $S_1$. That is, the modified supersaturation coefficient $S_2$ falls in the range between 0.6 and 1.4 times as much as the supersaturation coefficient $S_1$ normalized by a denominator that is a linear equation including $C_M/C_C$. The median value in this case is 1.0 times.

In this case, likewise, the corrector 103 illustrated in FIG. 1 applies the value of the magnesium ion concentration and the value of the calcium ion concentration both measured by the measurer 101 to the following formula 7, and calculates, from the supersaturation coefficient $S_1$, the modified supersaturation coefficient $S_2$ that is corrected taking into consideration the effect of the magnesium ion on the deposition rate of the calcium carbonate.

[Formula 7]

$$\frac{0.6S_1}{\{1 + 0.2(C_M / C_C)\}} \le S_2 \le \frac{1.4S_1}{\{1 + 0.2(C_M / C_C)\}}$$

Hence, according to the water quality checking that takes into consideration the effect of the magnesium ion concentration, first, the calcium ion concentration, the carbonate ion concentration, the hydrogen ion concentration, and the water temperature are measured, and the magnesium ion concentration is further measured. Subsequently, the supersaturation coefficient $S_1$ of the calcium carbonate out of consideration for any dissolved ions other than the calcium ion concentration, the carbonate ion concentration, and the hydrogen ion concentration is calculated. Next, correction is performed to obtain the modified supersaturation coefficient $S_2$ through the above formula 7 by applying the calculated value of the magnesium ion concentration and that of the calcium ion concentration. The determiner 104 illustrated in FIG. 1 determines the water quality based on this modified supersaturation coefficient $S_2$. Consequently, the water quality affecting the calcium scale build-up in the water flowing in the heat exchanger of the hot-water feeder system, and the like can be checked more precisely.

First Embodiment

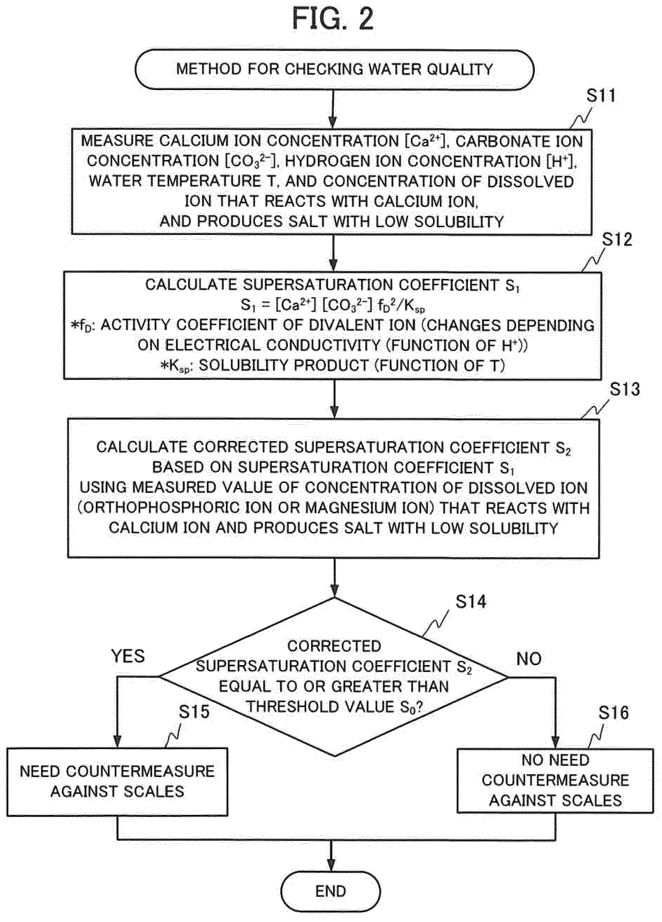

FIG. 2 is a flowchart illustrating a method for checking a water quality according to a first embodiment. As illustrated in FIG. 2, in the water quality checking, first, respective values of the calcium ion concentration, the carbonate ion concentration, the hydrogen ion concentration, the water temperature, and the concentration of dissolved ion (the orthophosphoric ion concentration, the magnesium ion concentration, or the like) that reacts with calcium ion, and that produces salt with low solubility are measured (step S11). Next, the supersaturation coefficient $S_1$ is calculated (step S12) based on the measured values of the calcium ion concentration, carbonate ion concentration, hydrogen ion concentration, and water temperature.

Subsequently, the modified supersaturation coefficient $S_2$ is calculated (step S13) from the supersaturation coefficient $S_1$. In this case, it is determined (step S14) whether or not the modified supersaturation coefficient $S_2$ is equal to or greater than a threshold value $S_0$. When the modified supersaturation coefficient $S_2$ is equal to or greater than the threshold value $S_0$ (step S14; YES), it can be determined that the calcium scale build-up will further advance, thereby determining (step S15) that a countermeasure against scales is necessary. When the modified supersaturation coefficient $S_2$ is smaller than the threshold value $S_0$ (step S14; NO), it can be determined that the calcium scale build-up will not further advance, thereby determining (step S16) that the countermeasure against scales is not necessary.

Although it is described later in more detail, the threshold value $S_0$ is a value defined in accordance with a hot-water feeder to which the method for checking a water quality according to, for example, the first embodiment is utilized and applied. In the fourth to seventh embodiments discussed later, a hot-water feeder system to which the method for checking a water quality described in the first embodiment is applied is described in more detail. In the fourth to seventh embodiments, explanations are given of a hot-water feeder system that includes various water quality (checking) controllers that function and operate to suppress calcium scale build-up so as to decrease the modified supersaturation coefficient $S_2$ when it is determined that the modified supersaturation coefficient $S_2$ is equal to or greater than the threshold value $S_0$. However, for example, in the hot-water feeder system that includes a device that performs only the water quality checking process and step as discussed in the first embodiment, a separate water quality control device, a water quality improving device, or the like, may be designed as a detachable device.

Second Embodiment

In a second embodiment, an explanation is given of a method for checking a water quality that includes calculating an amount of scale build-up by applying the modified supersaturation coefficient $S_2$, and determining the water quality. According to this method, the precision of the method for checking a water quality of the aforementioned first embodiment can be further improved.

The deposition rate R of the calcium carbonate in a predetermined water quality that does not contain any dissolved ion other than calcium ion, carbonate ion, and hydrogen ion that reacts with calcium, and that produces salt with low solubility can be given in the same manner using aforementioned formula 1. In this case, when an induction time period $t_{ind}$ is a time until scales are deposited, the induction time period $t_{ind}$ is proportional to the reciprocal of aforementioned formula 1, and thus the following proportional relationship can be obtained.

[Formula 8]

$$\ln(t_{ind}) \propto \frac{\gamma^3}{T^3 (\ln S_1)}$$

Accordingly, a relationship among the supersaturation coefficient $S_1$, the induction time period $t_{ind}$, and an induction time period $t'_{ind}$ at the modified supersaturation coefficient $S_2$ in the water quality having the orthophosphoric ion concentration and the magnesium ion concentration taken into consideration can be given by the following formula.

[Formula 9]

$$\frac{t'_{ind}}{t_{ind}} = \frac{\exp(\dfrac{\gamma^3}{T^3(\ln S_2)})}{\exp(\dfrac{\gamma^3}{T^3(\ln S_1)})}$$

In this case, a value of the interfacial energy $\gamma$ between the deposition nucleus and water is obtained in advance. In addition, the induction time period $t_{ind}$ in the predetermined water quality that does not contain any dissolved ions, such as orthophosphoric ion and magnesium ion, which reacts with calcium ion, and which produces salt with low solubility is measured through the device. This allows a calculation of the induction time period $t'_{ind}$ of the water quality relating to the modified supersaturation coefficient $S_2$. As for the interfacial energy $\gamma$, the interfacial energy $\gamma$ of scales containing no phosphoric acid is 0.018 J/m$^2$. The interfacial energy $\gamma$ of scales that contain phosphoric acid is 0.024 J/m$^2$.

In addition, a growth speed U of scales can be calculated through the following formula using the supersaturation coefficient S where k represents a reaction rate coefficient that is a physical property value that changes in accordance with the temperature.

[Formula 10]

$$U = kS$$

When the water temperature differs, the above reaction rate coefficient k can be calculated through the following formula.

[Formula 11]

$$k = k_0 \exp\frac{\varepsilon_a}{RT}$$

In the formula, $\varepsilon_a$ is the activation energy that is a temperature-independent physical property value, and that is unique to a substance (see, for example, Wiecher et al., Water Research, 1975, 9(9): pp.835-845). The activation energy in the case of calcium carbonate is 10.3 kcal/mol. With the use of these values, where a water temperature is $T_1$, a supersaturation coefficient is $S_1$, and a growth speed is $U_1$, and where a water temperature is $T_2$, a modified supersaturation coefficient is $S_2$, and a growth speed is $U_2$, a relative value of growth speed $U_1$ and growth speed $U_2$ can be calculated through the following fonnula.

[Formula 12]

$$\frac{U_2}{U_1} = \frac{S_2 \exp(\dfrac{\varepsilon_a}{RT_2})}{S_1 \exp(\dfrac{\varepsilon_a}{RT_1})}$$

By applying the formula 12, it becomes possible to calculate, from the modified supersaturation coefficient $S_2$, calculated through the above-discussed formula 5, formula 6, and formula 7, the ratio between the scale growth speed $U_2$ in this case and the growth speed $U_1$ in the predetermined water quality that does not contain any dissolved ions, such as orthophosphoric ion and magnesium ion, which reacts with calcium ion, and which produces salt with low solubility. Hence, by having measured the induction time period $t_{ind}$ and the growth speed $U_1$ in the water quality through the device with the supersaturation coefficient $S_1$ that is out of consideration for orthophosphoric ion concentration, and the like, and further scale build-up amount $V_1$ during a specific time period, the induction time period $t'_{ind}$ and the growth speed $U_2$ at the modified supersaturation coefficient $S_2$ can be calculated.

The induction time period is a time until scale deposition starts, the growth speed is a speed that the deposited scales grow, and the scale build-up amount is proportional to the growth speed after the deposition starts. Hence, through checking, a scale build-up amount $V_2$ at an arbitrary time (for example, an expected use period) in the water quality having taken into consideration orthophosphoric ion concentration and magnesium ion concentration can be calculated. According to the method for checking a water quality of the second embodiment, the water quality is determined by determining the scale build-up amount $V_2$ at an arbitrary time that is calculated as discussed above.

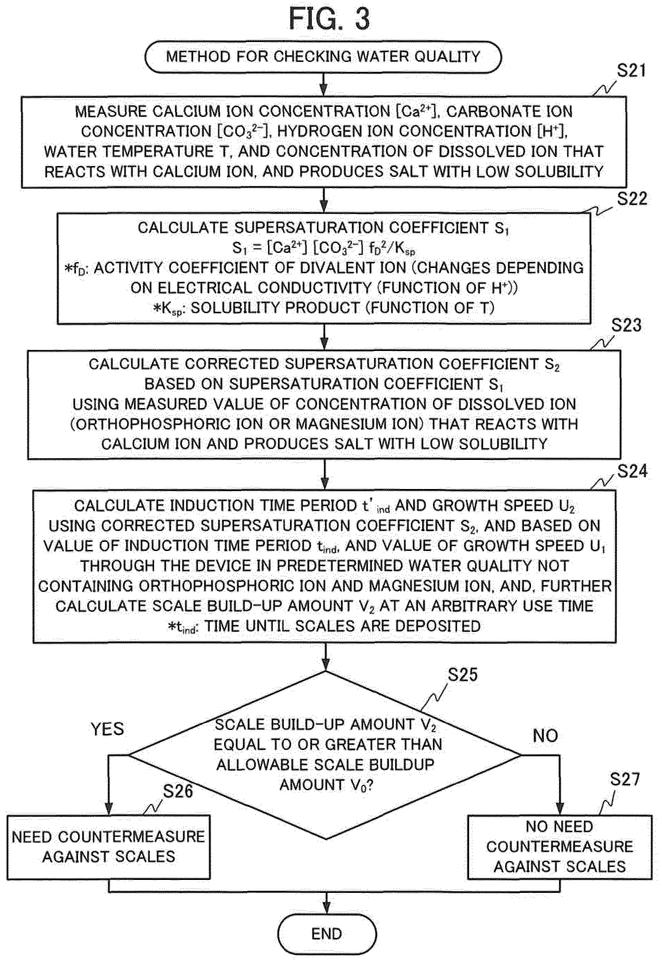

FIG. 3 is a flowchart illustrating the method for checking a water quality according to the second embodiment. As illustrated in FIG. 3, a step S21, a step S22, and a step S23 up to the calculation of the modified supersaturation coefficient $S_2$ are the same as step S11, step S12, and step S13, respectively, as discussed above in the first embodiment.

Subsequent to step S23, the induction time period $t'_{ind}$ and the growth speed $U_2$ are calculated through the aforementioned method, that is, by applying the modified supersaturation coefficient $S_2$ based on the respective values of the induction time period $t_{ind}$ and the growth speed $U_1$ through the device in a predetermined water quality not containing any dissolved ion, such as orthophosphoric ion and magnesium ion which reacts with calcium ion, and which produces salt with low solubility. In addition, the scale build-up amount $V_2$ at an arbitrary use period is calculated (step S24). Next, whether or not the scale build-up amount $V_2$ is equal to or greater than an allowable scale build-up amount $V_0$ is determined (step S25).

When the scale build-up amount $V_2$ is equal to or greater than the allowable scale build-up amount $V_0$ (step S25; YES), a functional deterioration caused by scale build-up is expected, thus it is determined (step S26) that a countermeasure against scales is necessary. When the scale build-up amount $V_2$ is smaller than the allowable scale build-up amount $V_0$ (step S25; NO), the expected functional deterioration caused by scale build-up is within an allowable range, thus it is determined (step S27) that a countermeasure against scales is unnecessary. The allowable scale build-up amount $V_0$ is a value that specifies for the same purpose as the threshold value $S_0$ of the first embodiment, and is, for example, a value defined as needed in accordance with a hot-water feeder to which the method for checking a water quality according to the second embodiment is utilized and applied.

Third Embodiment

In a third embodiment, an explanation is given of a method for checking a water quality that does not involve the determination of a water quality based on the scale build-up amount $V_2$ as performed in the aforementioned second embodiment, but includes determining the water quality through a calculation of a time at which a countermeasure against scales, or cleaning or replacement of the heat exchanger becomes necessary, and utilizing this calculated time as an indicator.

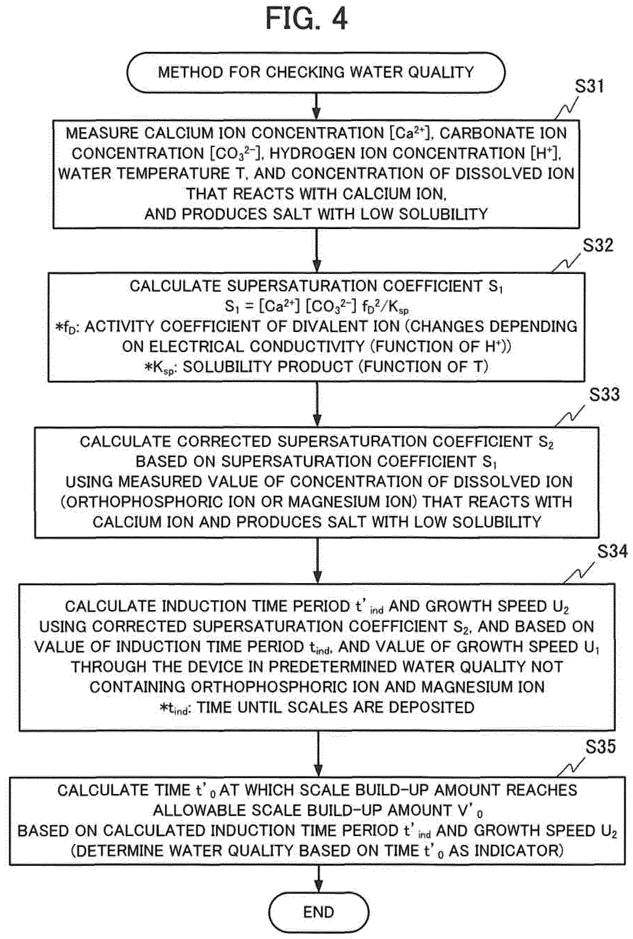

FIG. 4 is a flowchart illustrating a method for checking a water quality according to the third embodiment. As illustrated in FIG. 4, a step S31, a step S32, and a step S33 up to the calculation of the modified supersaturation coefficient $S_2$ are the same as step S11, step S12, and step S13, respectively, as discussed above in the first embodiment.

Subsequent to step S33, the induction time period $t'_{ind}$ and the growth speed $U_2$ are calculated (step S34) through the aforementioned method of the second embodiment, that is, by applying the modified supersaturation coefficient $S_2$ based on the respective values of the induction time period $t_{ind}$ and the growth speed $U_1$ through the device in predetermined water quality not containing any dissolved ion, such as orthophosphoric ion and magnesium ion which reacts with calcium ion, and which produces salt with low solubility. Next, a time (indicated as $t'_0$) at which the scale build-up amount of the water quality that is subjected to checking reaches an allowable scale build-up amount $V'_0$ (the scale build-up amount that indicates the necessity of a countermeasure against scales, or cleaning or replacing of the heat exchanger) is calculated based on the calculated induction time period $t'_{ind}$ and growth speed $U_2$. The water quality is then determined (step S35) based on the indicator that is the time $t'_0$ at which the countermeasure against scales, or cleaning or replacement of the heat exchanger becomes necessary. When, for example, the time $t'_0$ is shorter than a normally expected use period for the heat exchanger, it can be determined that the water quality requires the countermeasure against scales, or cleaning or replacement action for the heat exchanger. Conversely, when the time $t'_0$ is longer than the expected use period, it can be determined that the water quality does not require any countermeasure against scales, or cleaning or replacement of the heat exchanger for a long period of time.

The calculated time $t'_0$ that is a time at which the countermeasure against scales, or cleaning or replacement of the heat exchanger becomes necessary may be, for example, designed such that this calculated time is displayed on the water quality checking device or the hot-water feeder to which the method for checking a water quality according to the third embodiment is utilized or applied. The allowable scale build-up amount $V'_0$ (the scale build-up amount that indicates the necessity of a countermeasure against scales, or cleaning or replacement of the heat exchanger) is a value that is, for example, defined as needed in accordance with a hot-water feeder to which the method for checking a water quality of the third embodiment is utilized or applied in the same way as the defined value as discussed above in the first embodiment and the second embodiment.

Fourth Embodiment

In a fourth embodiment and the subsequent embodiments, actual forms of the hot-water feeder is described in more detail. In the aforementioned first embodiment to third embodiment, each of the different means (step S14, step S25, or step S35) at the stage of the determination means for the necessity of the countermeasure against scales, or of cleaning or replacement of the heat exchanger was discussed. In the following explanation, the determination means in the step S14 of the aforementioned first embodiment is representatively explained. Applications of other determination means to the hot-water feeder system will be apparent to those skilled in the art in view of the following descriptions.

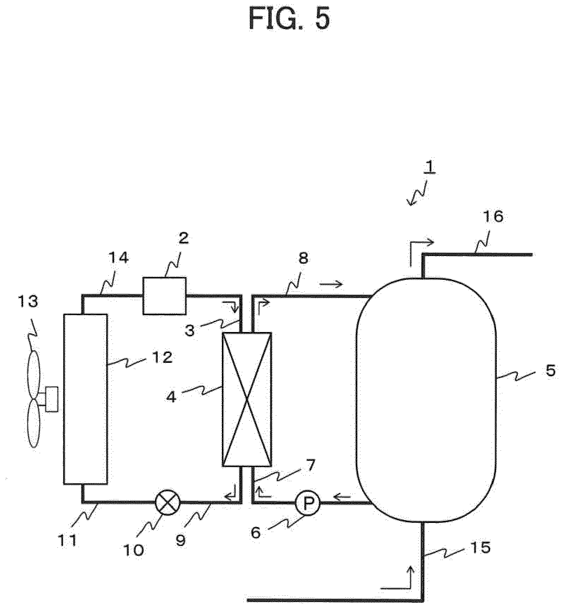

FIG. 5 is a schematic diagram illustrating a structure of a coolant circuit and that of a water circuit both in a heat pump heat-exchanging type hot-water feeder system. As illustrated in FIG. 5, a typical hot-water feeder system 1 includes a compressor 2, a first coolant piping 3, a coolant/water heat exchanger 4, a hot water tank 5, a pump 6, a first water piping 7, a second water piping 8, a second coolant piping 9, an expansion valve 10, a third coolant piping 11, an evaporator 12, a fan 13, a fourth coolant piping 14, a first water supply piping 15, and a second water supply piping 16.

First, a typical heat pump heat-exchanging type hot-water feeder system is explained with reference to FIG. 5. A heat-gas coolant (for example, carbon dioxide, hydrofluorocarbon, or the like) that has become high-temperature and high-pressure by the compressor 2 flows through the first coolant piping 3, and flows into the coolant/water heat exchanger 4. Water that is stored in the hot water tank 5 is pushed out by the pump 6, flows through the first water piping 7, and flows into the coolant/water heat exchanger 4. The coolant/water heat exchanger 4 heats water by heat exchange between the heat-gas coolant and the water. The heated water flows through the second water piping 8, and returns to the hot water tank 5.

The heat-gas coolant that has transferred the heat to the water flows through the second coolant piping 9, and is delivered to the expansion valve 10. The heat-gas coolant that has been delivered to the expansion valve 10 is decompressed, flows through the third coolant piping 11, and flows into the evaporator 12. The evaporator 12 absorbs heat through the outside air delivered by the fan 13, and the heat-gas coolant flows through the fourth coolant piping 14, and returns to the compressor 2. The heated water is supplied to a use point from the second water supply piping 16.

For example, in the case of a typical heat pump hot-water feeder system that boils water by utilizing midnight electric power, when hot water in the hot water tank 5 is used during the daytime, tap water is supplied from the first water supply piping 15 to the hot water tank 5 in accordance with a usage amount of hot water. In the hot water tank 5, as long as there is no stirring, the water is separated into two layers that are a high temperature layer and a low temperature layer, and thus the supply of tap water causes an interfacial layer portion between the high temperature layer and the low temperature layer to move up (to a water-outflow side of the hot water tank 5) in the hot water tank 5. Hence, high temperature hot water can always be supplied during the daytime. In this case, when the typical heat pump hot-water feeder system is operated with water having a level of quality at which scales build up, the scales build up on the heat-transfer surface of the water circuit in the coolant/water heat exchanger 4 on the water outflow side.

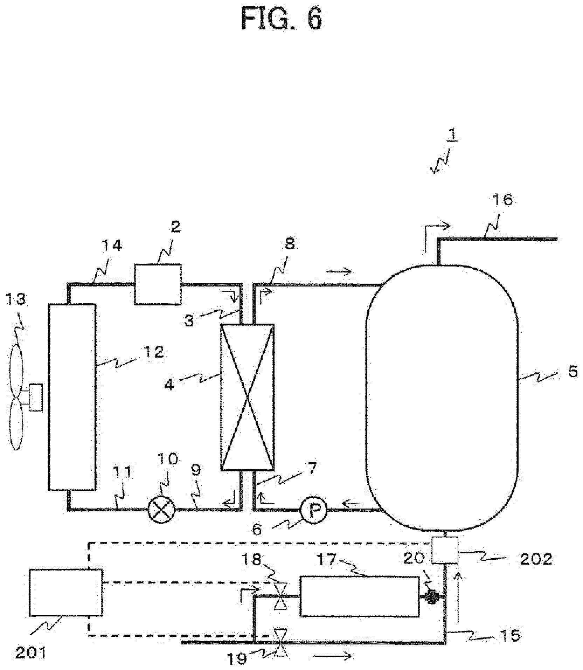

FIG. 6 is a schematic diagram illustrating a structure of a hot-water feeder system according to the fourth embodiment of the present invention. Although the basic structural elements are the same as those in FIG. 5, as illustrated in FIG. 6, the hot-water feeder system 1 of the fourth embodiment further includes a water quality controller 17, a first flow-rate control valve 18, a second flow-rate control valve 19, a conductivity meter 20, a water quality checking controller 201, and a water quality measurer 202.

As illustrated in FIG. 6, tap water that is supplied from the first water supply piping 15 to the hot water tank 5 is a merged water flow that is water flowing from the water quality controller 17 with the water flow volume adjusted by the first flow-rate control valve 18, and water directly flowing in without flowing through the water quality controller 17 and with the water flow volume adjusted by the second flow-rate control valve 19. By adjusting the water flow volume of the water flowing through the water quality controller 17, a mechanism of improving the water quality of water flowing into the hot water tank 5 is accomplished.

More specifically, when it is determined that the water quality can build up scales based on data from the water quality measurer 202, a part of the supply water is caused to flow through the water quality controller 17 in order for the water flowing into the hot water tank 5 to stay within a reference value for an item that was out of the reference value, thereby suppressing scale build-up. At this time, the water quality checking controller 201 controls the open and close conditions of the first flow-rate control valve 18 and those of the second flow-rate control valve 19.

The water quality measurer 202 includes a pH electrode, an electrical-conductivity electrode, a phosphate ion electrode, a calcium ion electrode, a carbonate ion electrode, a thermocouple, and the like. The water quality measurer is a converter that converts an output signal from each electrode or the thermocouple to a numerical value in each water quality item, and generates an analog or digital signal to control the water quality checking controller 201.

In this case, a specific example of the water quality controller 17 is a cartridge or the like filled with a water purification resin represented by an ion exchange resin, or, a substance that provides sustained-release of orthophosphoric ion or magnesium ion.

An example water purification resin applicable is a polymer having a basic structure of styrene-divinylbenzene copolymer. In general, the water purification resin is known to have a function that selectively exchanges cation when having an acidic functional group, and selectively exchanges anion when having a basic functional group. For example, a value of the modified supersaturation coefficient $S_2$ calculated by the water quality checking controller 201 of the fourth embodiment becomes greater as the calcium ion concentration, and the carbonate ion concentration become larger, and the value of the modified supersaturation coefficient becomes greater as the orthophosphoric ion concentration becomes smaller. Thus, when, for example, tap water contains orthophosphoric ion, it is desirable to remove calcium ion using a cation exchange resin only, and to allow the orthophosphoric ion to pass through. In particular, a resin that has sulfonic acid group as an exchange group is more desirable since efficient removal of calcium ion can be achieved.

Conversely, when tap water contains no orthophosphoric ion, it is desirable to utilize both cation exchange resin and anion exchange resin to remove calcium ion and carbonate ion. In addition, a resin that has an amino group introduced as the functional group of the water purification resin is more desirable since such resin can efficiently remove carbonate ion.

When, however, the lifetime with regard to the ion exchange performance comes to an end, calcium ion and bicarbonate ion cannot be easily captured any more, and the ion exchange with hydrogen ion and hydroxide ion can no longer be performed. To detect this condition, the conductivity meter 20 may monitor the conductivity of the processed water. For example, a replacement time of the water purification resin can be notified when the conductivity meter 20 has an alarm function and is set to give an alert if the conductivity exceeds 10 $\mu$S/cm. In addition, a measured value of the conductivity meter 20 may be transmitted to the water quality checking controller 201, and the water quality checking controller 201 may give an alert.

Note that an example desirable substance that is filled in the cartridge and that provides sustained-release of orthophosphoric ion is a salt that contains orthophosphoric acid-based ion in the crystal, such as $FePO_4 \cdot 2H_2O$, $AlPO_4 \cdot 2H_2O$, $CaHPO_4$, $Ca_4H(PO_4)_3$, $Ca_{10}(PO_4)_6(OH)_2$, $Ca_{10}(PO_4)_6F_2$, $CaHAL(PO_4)_2$, $CaF_2$, $MgNH_4PO_4$, $FeNH_4PO_4$, or $Fe_2(PO_4)_2$. The shape of the substance that provides sustained-release of the orthophosphoric ion can be selected among a cylindrical shape, a spherical shape, a hallow shape, a fiber shape, and the like, and it is desirable to have a microscopic structure with a contact area of equal to or greater than 1 $m^2$ per 1 $m^3$ water.

An example desirable substance that is filled in the cartridge, and that provides sustained-release of magnesium ion is a salt that contains magnesium ion in the crystal, such as $Mg(OH)_2$, $MgSO_4$, $CaMg(CO_3)_2$, $Mg_3Si_2O_5(OH)_4$, $Mg_2Si_3O_{7.5}(OH) \cdot 3H_2O$, $Mg_3Si_4O_{10}(OH)_2 \cdot H_2O$, or $MgNH_4PO_4$. Likewise the case of the orthophosphoric ion, a shape of the substance that provides sustained-release of the magnesium ion can be selected among a cylindrical shape, a spherical shape, a hallow shape, a fiber shape, and the like, and it is desirable to have a microscopic structure with a contact area of equal to or greater than 1 $m^2$ per 1 $m^3$ water.

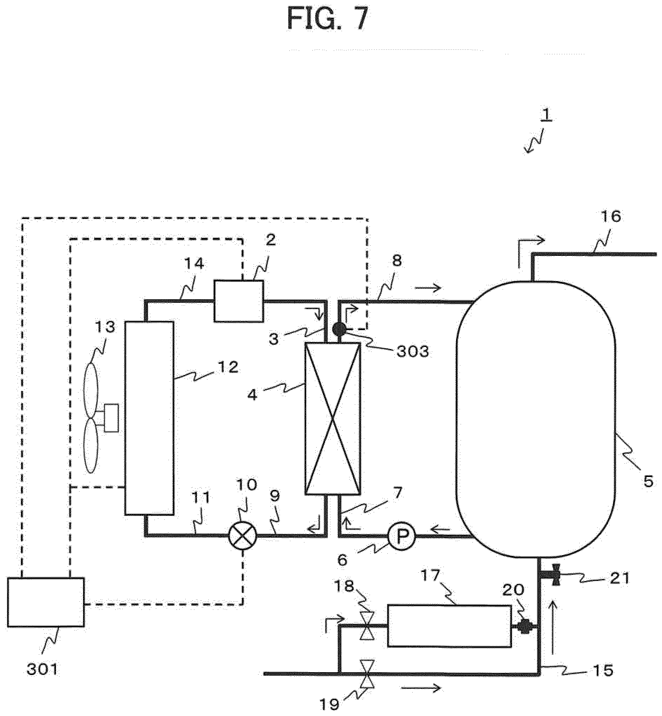

According to the hot-water feeder system 1 of the fourth embodiment, the water quality of water drawn from a water quality checking faucet (not shown in FIG. 6, but refer to a fifth embodiment and FIG. 7 discussed later) located inside the water quality measurer 202 is checked by applying the method for checking a water quality of the aforementioned first embodiment to third embodiment. Furthermore, the water quality of water that is supplied based on this result is controlled. More specifically, the measurement, the calculation, the correction, the determination, and the like are performed by the water quality checking controller 201, and the water quality measurer 202 both illustrated in FIG. 6. In more detail, first of all, the supersaturation coefficient $S_1$ is calculated based on respective measured values of the calcium ion concentration, the carbonate concentration, and the hydrogen ion concentration, and, a set water temperature inside the hot water tank 5. Subsequently, the supersaturation coefficient $S_1$ is corrected to $S_2$ using the measured value of the orthophosphoric ion concentration (or the magnesium ion concentration, or the like) contained in a solution. When the modified supersaturation coefficient $S_2$ that has been corrected is equal to or greater than the threshold value $S_0$, it is determined that the calcium scale build-up on the coolant/water heat exchanger 4 will further advance.

When a determination reference is not satisfied (when it is determined that the calcium scale build-up will further advance), the water flow volume at the first flow-rate control valve 18 is adjusted so as to increase the water flow volume at the water quality controller 17, and the water quality of the water drawn from the water quality checking faucet is checked again. When the determination reference is not satisfied again, the water flow volume at the first flow-rate control valve 18 is adjusted so as to increase the water flow volume at the water quality controller 17. By repeating such adjustments, the water quality that does not build-up any scales can be obtained. Note that the same adjustment may be performed by reducing the water flow volume at the second flow-rate control valve 19, or by adjusting both the first flow-rate control valve 18 and the second flow-rate control valve 19.

The set value $S_0$ to be compared with the modified supersaturation coefficient $S_2$ that has been corrected can be defined in accordance with an operation condition, water quality, and the frequency of maintenance of the hot-water feeder. In general, when regular maintenance is not performed for equal to or longer than 15 years, it is desirable that the water quality improving system should be operated with the set value of $S_0 = 1$. When regular maintenance is performed every five years, it is desirable that the water quality improving system should be operated with the set value of $S_0 = 6$. When regular maintenance is performed every year, it is desirable that the water quality improving system should be operated with the set value of $S_0 = 12$.

As discussed above, the hot-water feeder system 1 according to the fourth embodiment operates the water quality control means and the calcium scale build-up suppression means based on a further precise and accurate water quality checking result of the heated water, thereby achieving an effect of surely suppressing scale build-up on the heat transfer surface of the coolant/water heat exchanger 4.

Fifth Embodiment

FIG. 7 is a schematic diagram illustrating a structure of a hot-water feeder system according to a fifth embodiment of the present invention. As illustrated in FIG. 7, the hot-water feeder system 1 of the fifth embodiment employs a structure in which additional structural elements that are a water quality checking controller 301, and a water temperature sensor 303 are added to the hot-water feeder system 1 of the aforementioned fourth embodiment. Note that, in FIG. 7, except a water quality checking faucet 21, illustration of the structure of the water quality checking controller 201 and that of the water quality measurer 202 are omitted.

The hot-water feeder system 1 of the fifth embodiment includes the water quality checking controller 301 that adjusts, based on a signal from the water temperature sensor 303 inside the second water piping 8, the rotation speed of the compressor 2, the rotation speed of the fan 13 and/or the gap of a flow passage of the expansion valve 10. This structure is intended to control the water temperature inside the second water piping 8.

When, for example, a temperature is to be decreased, an operation that slows down the rotation speed of the compressor 2 and that of the fan 13, and increases the gap of the flow passage of the expansion valve 10 is performed. The water quality checking controller 301 includes an interface into which a supersaturation coefficient is input, and performs the above-described control based on the input coefficient. The water quality checking controller 301 may be integrated with the water quality checking controller 201 (not shown) as discussed above in the fourth embodiment. In such a case, the water quality checking controller 301 may directly calculate, correct, and the like, the supersaturation coefficient $S_1$ and the modified supersaturation coefficient $S_2$.

In general, in order to maximize a potential heat quantity in the hot water tank 5, it is effective to maximize the water temperature at the outflow side of the evaporator 12, and such a temperature is often and normally set between 60 °C and 90 ° C. However, the higher the temperature is, the more calcium carbonate is likely to be deposited, and thus the supersaturation coefficient $S_1$ to a certain solution composition becomes large as the water temperature becomes high. That is, according to the method for checking a water quality of the aforementioned first embodiment to third embodiment, the greater the supersaturation coefficient $S_1$ calculated based on the calcium ion concentration, the carbonate ion concentration, the hydrogen ion concentration, and the water temperature becomes, the greater the modified supersaturation coefficient $S_2$ that has been corrected becomes in proportion to the supersaturation coefficient $S_1$.

In the fifth embodiment, the water quality of water drawn from the water quality checking faucet 21 for the supply water provided at the first water supply piping 15 is also checked by the water quality checking controller 301 (or the water quality checking controller 201 (not shown)) to which the aforementioned method for checking a water quality is applied. However, according to the method for checking a water quality of the fifth embodiment, the supersaturation coefficient $S_1$ is calculated based on the respective measured values of the calcium ion concentration, carbonate ion concentration, and hydrogen ion concentration in the drawn water, and the measured value of the water temperature through the water temperature sensor 303 inside the second water piping 8.

In addition, the supersaturation coefficient $S_1$ is corrected to $S_2$ using the measured value of the orthophosphoric ion concentration (or magnesium ion concentration) contained in a solution, and when the modified supersaturation coefficient $S_2$ that has been corrected is equal to or greater than the threshold value $S_0$, it is determined that the calcium scale build-up on the water/coolant heat exchanger 4 will further advance.

When the determination reference is not satisfied (when it is determined that the calcium scale build-up will further advance), first, the water temperature inside the second water piping 8 is calculated in order to decrease the modified supersaturation coefficient $S_2$ that has been corrected through the method for checking a water quality of the aforementioned first embodiment to be smaller than the threshold value $S_0$. Next, by adjusting the rotation speed of the compressor 2, the rotation speed of the fan 13, and/or the gap of the flow passage of the expansion valve 10 through the water quality checking controller 301, the water temperature inside the second water piping 8 is set to a predetermined temperature.

Like the aforementioned fourth embodiment, the set value $S_0$ to be compared with the modified supersaturation coefficient $S_2$ that has been corrected can be defined depending on an operation condition, water quality, and the frequency of maintenance of the hot-water feeder. In general, when regular maintenance is not performed for equal to or longer than 15 years, it is desirable that the water quality improving system should be operated with the set value of $S_0 = 1$. When regular maintenance is performed every five years, it is desirable that the water quality improving system should be operated with the set value of $S_0 = 6$. When regular maintenance is performed every year, it is desirable that the water quality improving system should be operated with the set value of $S_0 = 12$.

When the water temperature at the outflow side of the water/coolant heat exchanger 4 decreases, the potential heat quantity in the hot water tank 5 decreases, causing a problem whereby, for example, the hot water runs out. Hence, when the water temperature inside the second water piping 8 is controlled by the water quality checking controller 301, it is desirable to set the water temperature to be between 40 °C and 90 °C.

As discussed above, the hot-water feeder system 1 according to the fifth embodiment operates the water quality control means and the calcium scale build-up suppression means based on a further precise and accurate water quality checking result of the heated water, thereby achieving an effect of surely suppressing scale build-up on the heat transfer surface of the coolant/water heat exchanger 4. In addition, when applied together with the hot-water feeder system 1 of the aforementioned fourth embodiment, the effect of suppressing scale build-up on the heat transfer surface can be further surely accomplished.

Sixth Embodiment

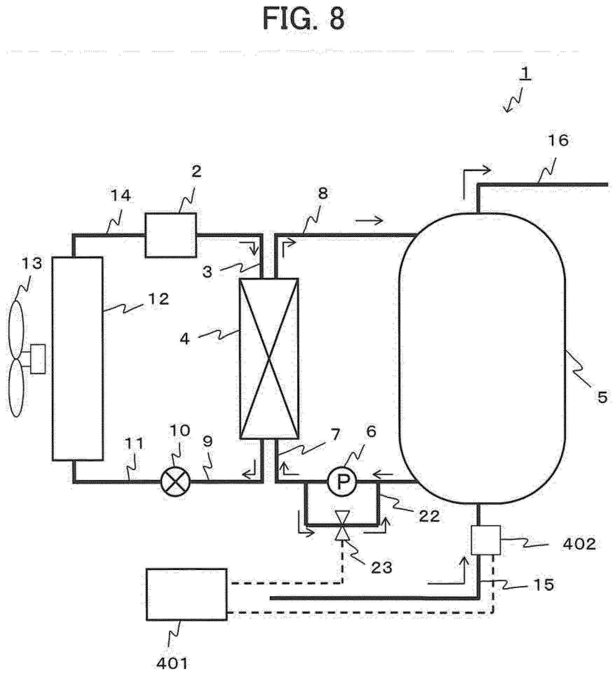

FIG. 8 is a schematic diagram illustrating a structure of a hot-water feeder system according to a sixth embodiment of the present invention. As illustrated in FIG. 8, the hot-water feeder system 1 of the sixth embodiment includes a bypass circuit piping 22, an automatic open/close valve 23, a water quality checking controller 401, and a water quality measurer 402 (includes thereinside a water quality checking faucet) in addition to the structural elements of the typical hot-water feeder system 1 illustrated in FIG. 5.

In the aforementioned fourth embodiment and fifth embodiment, a specific structure is disclosed for the device that controls the modified supersaturation coefficient $S_2$ that has been corrected to be smaller than the threshold value $S_0$ so as to control the scale build-up on the heat transfer surface through the water quality control means or the water temperature control means when it is determined that the operation condition causes, based on the result of the water quality checking, scale build-up. According to the hot-water feeder system 1 of the sixth embodiment, scale build-up on the coolant/water heat exchanger 4 is suppressed by controlling the water flow volume that flows through the first water piping 7 and the second water piping 8 even if the modified supersaturation coefficient $S_2$ that has been corrected is equal to or greater than the threshold value $S_0$.

More specifically, as illustrated in FIG. 8, the bypass circuit piping 22 is provided to connect an inflow side of the pump 6 and an outflow side of the pump that delivers water to the coolant/water heat exchanger 4, and the automatic open/close valve 23 is installed on this bypass circuit piping 22 to open and close the valve at a regular cycle. Consequently, pulsating flow is produced in water delivered to the coolant/water heat exchanger 4, thereby suppressing scale build-up. The water quality check is performed by the water quality checking controller 401 and the water quality measurer 402 in the same way as those of the aforementioned embodiments (in particular, the fourth embodiment), and the opening and closing of the valve is controlled and adjusted as needed based on this check result.

An example pulsation flow pattern applicable is a sine wave, a square wave, a triangular wave, or the like, and it is desirable to have the flow rate periodically changed between a maximum flow rate $Q_1$ and a minimum flow rate $Q_2$. A time $T_1$ (the width of pulsation flow) of the flow rate $Q_1$ is desirably within a range between 0.1 and 10 seconds, and a pulsation flow frequency $T_2$ is desirably adjusted to be within a range of one to twenty times as much as the pulsation flow width $T_1$.

An optimum condition for the pulsation flow varies depending on the water quality of utilized water, and the condition of the hot water supply. When an outflowing hot water temperature is between 40 °C and 60 ° C, and the water quality has the modified supersaturation coefficient $S_2$ that has been corrected at room temperature and that is equal to or less than 2, it is desirable to define the ratio of the maximum flow rate $Q_1$/the minimum flow rate $Q_2$ to be within a range between one and two. When the water quality has the modified supersaturation coefficient $S_2$ that has been corrected at room temperature and that is equal to or greater than two, it is desirable to define the ratio of the maximum flow rate $Q_1$/the minimum flow rate $Q_2$ to be within a range between one and four for the water quality that is likely to produce calcium carbonate. In addition, when water that has the modified supersaturation coefficient $S_2$ that has been corrected at room temperature and that is equal to or greater than two is heated to 60 °C to 90 ° C, it is desirable to define the ratio of the maximum flow rate $Q_1$/the minimum flow rate $Q_2$ to be within the range between one and six.

As discussed above, the hot-water feeder system 1 of the sixth embodiment creates pulsation flow in water that is delivered to the coolant/water heat exchanger 4 based on a further precise and accurate water quality checking result to add a change in flow speed toward the water-side heat transfer surface of the coolant/water heat exchanger 4, thereby suppressing build-up of the deposited scales.

Seventh Embodiment

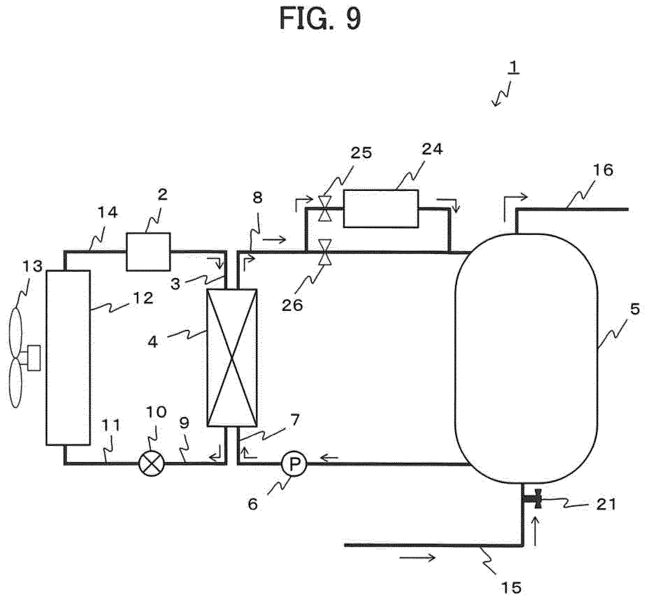

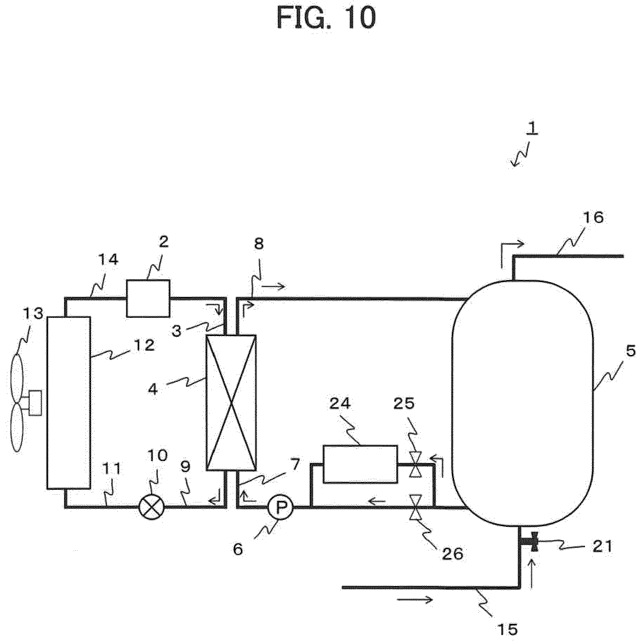

FIG. 9 is a schematic diagram illustrating a structure of a hot-water feeder system according to a seventh embodiment of the present invention. In addition, FIG. 10 is a schematic diagram illustrating a structure of the hot-water feeder system according to a modified example of the seventh embodiment. As illustrated in FIG. 9 and FIG. 10, the hot-water feeder system 1 of the seventh embodiment is in a form that includes additional structural elements that are a scale particle catcher 24, a third flow-rate control valve 25, and a fourth flow-rate control valve 26 added to the hot-water feeder system 1 of the aforementioned fourth embodiment.

Note that in FIG. 9 and FIG. 10, except the water quality checking faucet 21, illustration of the respective structures of the water quality controller 17, the first flow-rate control valve 18, the second flow-rate control valve 19, the conductivity meter 20, the water quality checking controller 201, and the water quality measurer 202 that are the structural elements of the hot-water feeder system 1 of the fourth embodiment are omitted.

According to the seventh embodiment, by installing the scale particle catcher 24 to a flow passage constructed by the first water piping 7 or the second water piping 8, in addition to the effect of the hot-water feeder system 1 of the aforementioned fourth embodiment, calcium carbonate scale particles that are produced in the first water piping 7 and the second water piping 8 can be caught.

As illustrated in FIG. 9 and FIG. 10, the water flow volume that flows through the scale particle catcher 24 is adjusted as needed by the third flow-rate control valve 25, and the water flow volume that does not flow through the scale particle catcher 24 is adjusted by the fourth flow-rate control valve 26 as needed, improving the water quality of tap water supplied from the first water piping 7 or the second water piping 8 between the hot water tank 5 and the coolant/water heat exchanger 4. Adjustments by the third flow-rate control valve 25 and fourth flow-rate control valve 26 are, for example, performed by the water quality checking controller 201 and the water quality measurer 202 (not shown). Although the action and effect of the scale particle catcher 24 illustrated in FIG. 9 and those illustrated in FIG. 10 are the same, the water temperature of water flowing into the scale particle catcher 24 in FIG. 9 is higher than that of water flowing into the scale particle catcher 24 in FIG. 10 by 10 °C to 40 ° C. Hence, the catching performance by the scale particle catcher 24 in the structure of the hot-water feeder system 1 in FIG. 9 increases by a factor of 1.2 to 2.

A mechanism of building up scales on the water/coolant heat exchanger 4 includes either a case in which the scales grow directly on the heat transfer surface of the water/coolant heat exchanger 4, or a case in which microparticle scales produced in the water of the first water piping 7 and the second water piping 8, or of the hot water tank 5 are built up on the heat transfer surface of the water/coolant heat exchanger 4. The scale particle catcher 24 of this seventh embodiment removes microparticle scales that are produced in the water due to the latter cause, and suppresses a microparticle scale build-up on the water/coolant heat exchanger 4.

An example of the material of the filtrating body that is filled inside the scale particle catcher 24 is, copper, brass, stainless steel, silicone rubber, glass, iron, iron oxide (III, II), polytetrafluoroethylene (Teflon (registered trademark) resin (PTFE, PFA)), polyvinyl chloride, polyethylene, polystyrene, polypropylene, polysulfone, isoprene rubber, butadiene rubber, styrene-butadiene rubber, aromatic polyamide (nylon 6, nylon 6-6, or the like), or the like. Among these materials, any one of the following is preferable: copper, brass, stainless steel, silicone rubber, glass, iron, iron oxide (III, II), polyvinyl chloride, polyethylene, polystyrene, polypropylene, polysulfone, isoprene rubber, butadiene rubber, and styrene-butadiene rubber.

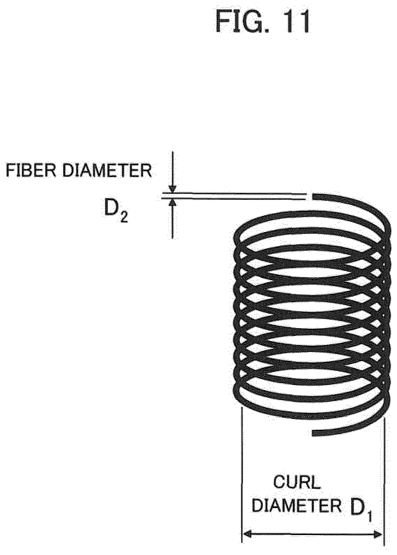

FIG. 11 is a diagram illustrating a curled fiber in a structure of scale filtrating body. For example, a cluster formed of curled fibers each having a curl diameter of $D_1$, and a fiber diameter of $D_2$ may be utilized inside the scale particle catcher 24. Practically, tensile strength between 2 and 4 kg is desirable for the curled fibers. In the hot-water feeder system 1 that heats water to be equal to or greater than 60 ° C, this condition corresponds to corrosion durability of equal to or longer than 10 years. As for curled fibers made of stainless steel, the fiber diameter $D_2$ even as long as equal to or greater than 10 $\mu$m can be utilized. It is desirable to set a volume $V_1$ of the occupying filler inside the scale particle catcher 24 to be within a range of $V_2/V_1 = 10$ to $500$ with reference to an internal volume $V_2$ of the hot water tank 5.

The water quality check is carried out using the water drawn from the water quality checking faucet 21 for the supply water provided at the first water supply piping 15, and by applying the methods according to the aforementioned first embodiment to third embodiment. According to the hot-water feeder 1 of the seventh embodiment, the supersaturation coefficient $S_1$ is calculated by the water quality checking controller 201 and the water quality measurer 202 (not shown) based on the respective measured values of the calcium ion concentration, carbonate ion concentration, and hydrogen ion concentration, and a set water temperature inside the hot water tank 5.

The supersaturation coefficient $S_1$ is corrected to $S_2$ using the measured value of the orthophosphoric ion concentration (magnesium ion concentration, or the like) contained in a solution, and when the modified supersaturation coefficient $S_2$ that has been corrected is equal to or greater than the threshold value $S_0$, it is determined that the calcium scale build-up on the water/coolant heat exchanger 4 will further advance.

When it is determined that the calcium scale build-up will further advance, the water flow volume at the third flow-rate control valve 25 is adjusted to increase a water flow rate $Q_3$ of water flowing through the scale particle catcher 24, and some of the microparticle scales produced in the water are removed. Hence, the amount of scales that build up on the heat transfer surface of the water/coolant heat exchanger 4 can be reduced. In this, the water flow rate of water that flows through the fourth flow-rate control valve 26 is defined as $Q_4$. When a difference $(S_2 - S_0)$ between the modified supersaturation coefficient $S_2$ and the threshold value $S_0$ is equal to or greater than zero and less than five, it is desirable to adjust a ratio $Q_3/(Q_3 + Q_4)$ of the water flow volume to be within a range between 0 and 0.5. In addition, when the difference $(S_2 - S_0)$ between the modified supersaturation coefficient $S_2$ and the threshold value $S_0$ is equal to or greater than five and equal to or less than 11, it is desirable to adjust the ratio $Q_3/(Q_3 + Q_4)$ of the water flow volume to be within a range between 0.25 and 1.0.

According to a heat pump hot-water feeder system utilizing such calcium scale build-up suppression means, the set value $S_0$ to be compared with the modified supersaturation coefficient $S_2$ that has been corrected can be defined in accordance with an operation condition, water quality, and the frequency of maintenance of the hot-water feeder system 1. In general, when regular maintenance is not performed for equal to or longer than 15 years, it is desirable that the water quality improving system should be operated with the set value of $S_0 = 1$. When regular maintenance is performed every five years, it is desirable that the water quality improving system should be operated with the set value of $S_0 = 6$. When regular maintenance is performed every year, it is desirable that the water quality improving system should be operated with the set value of $S_0 = 12$.

As discussed above, the hot-water feeder system 1 of the seventh embodiment operates the water quality control means and multiple calcium scale build-up suppression means based on a further precise and accurate water quality checking result of the heated water, thereby surely achieving an effect of further suppressing scale build-up on the heat transfer surface of the coolant/water heat exchanger 4.

EXAMPLES

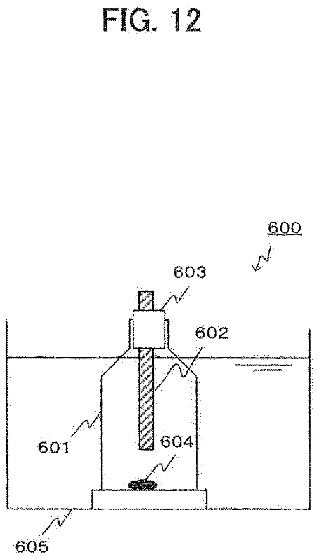

FIG. 12 is a diagram illustrating a structure of a scale deposition test device applied in an example. As illustrated in FIG. 12, a scale deposition test device 600 includes a heat-resistant glass vessel 601, an electrical-conductivity electrode 602, a silicone rubber 603, a magnetic stirrer 604, and a thermostatic bath 605.

More specifically, in a test, the electrical-conductivity electrode 602 (CT-27112B manufactured by TOA DKK) was inserted in the heat-resistant glass vessel 601 (volume: 585 mL) having an inner room cleaned with acid, and the heat-resistant glass vessel was gas-tightly sealed with the silicone rubber 603 so as to have a minimum gas phase. A sample water in the glass bottle was constantly stirred at substantially 300 rpm using the magnetic stirrer 604 formed of PTFE. Note that the water temperature of the sample water was adjusted by the thermostatic bath 605 to stay at a constant temperature that was 65 °C.

As soon as deposition of scales ($CaCO_3$) started during the test using the scale deposition test device 600, an aqueous solution turned slightly cloudy. After the deposition of $CaCO_3$, an endless number of $CaCO_3$ microparticles built up inside the heat-resistant glass vessel 601, and thus the heat-resistant glass vessel 601, the electrical-conductivity electrode 602, and the magnetic stirrer 604 were soaked in hydrochloric acid of 0.01 mol/L for equal to or longer than 20 minutes at room temperature to completely dissolve the microparticles. Note that prior to the heating of the sample water in the test, each aqueous solution was aerated by nitrogen gas (industrial nitrogen gas, purity: 99.995%, $CO_2$ concentration: equal to or less than 0.1 ppm) for five to 10 minutes to adjust pH to 8.4 to 8.7.

In general, $CaCO_3$ deposition reactions cause the ion concentration in a solution to be decreased, and thus the electrical conductivity of the solution decreases. For the purpose of quantization of the influence of phosphate concentration in the solution on the $CaCO_3$ deposition reaction, a time change in electrical conductivity of the solution was recorded using the scale deposition test device 600. More specific examples and results thereof are shown below.

First Example

In a first example, a test result obtained by using the scale deposition test device 600, performing a nitrogen gas aeration in advance as discussed above, and maintaining various sample waters with pH being adjusted from 8.4 to 8.7 at 65 °C for equal to or longer than several hundred hours are shown. The various sample waters that were used in the test were tap water (hereinafter, referred to as tap water) collected in Europe, a simulated aqueous solution (hereinafter, referred to as simulated water), an aqueous solution (first test water) that was the simulated water added with $K_3PO_4$ of 1 mg/L, and an aqueous solution (second test water) that was the simulated water added with $K_3PO_4$ of 5 mg/L.

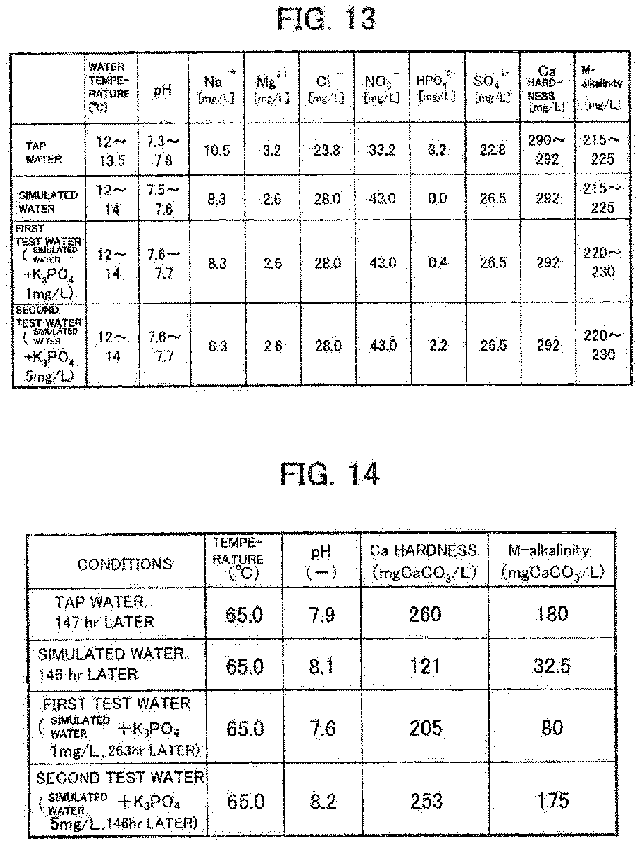

FIG. 13 is a diagram illustrating a water quality analysis result before heating according to the first example. When the tap water and the simulated water are compared, it becomes clear that the simulated water contained substantially the same amount of ions (some ions are not shown) with regard to major ions contained in tap water that are $Na^+$, $K^+$, $Ca^{2+}$, $Mg^{2+}$, $Cl^-$, $NO_3^-$, and $SO_4^-$, and that the pH of the simulated water was the same as that of the tap water. In addition, as for the $HPO_4^{2-}$ concentration of the aqueous solution (second test water) that was the simulated water added with $K_3PO_4$ of 5 mg/L, although it was 2.2 mg/L that was slightly lower than 3.2 mg/L contained in the tap water, the $HPO_4^{2-}$ concentration of this aqueous solution was substantially the same as that of the tap water.

FIG. 14 is a diagram illustrating a change in pH, Ca hardness, and M-alkalinity after heating according to the first example. Prior to heating, the pH was adjusted to 8.4 to 8.7 through the aeration of nitrogen gas, the pH of each sample water gradually decreased through the heating for several hundred hours, and eventually decreased to 7.6 to 8.2. This is presumably because that $CO_3^{2-}$ was consumed along with the deposition of calcium carbonate, causing a shift of equilibrium to the right for the dissociation reaction ($HCO_3^- \leftrightarrow H^+ + CO_3^{2-}$) of $HCO_3^-$, and thus $H^+$ was produced. In addition, the Ca hardness, and the M-alkalinity were also decreased along with the heating.

In each sample water, the supersaturation coefficient (a ratio of the amount of ions in a solution relative to the amount of ions in a saturation state) to calcium carbonate before heating was 112 to 165 times. After heating, however, the supersaturation coefficient was decreased to 1.7 to 3.4 times, and reached a substantially equilibrium state. Note that when X-ray diffraction of solid particles deposited in the aqueous solution was evaluated, calcium carbonate ($CaCO_3$) was contained but no calcium phosphate ($Ca_4(PO_4)_3$) was contained.

In this case, when the tap water and the aqueous solution (second test water) that was the simulated water added with $K_3PO_4$ of 5 mg/L are compared as illustrated in FIG. 14, the diminution of the pH and that of the Ca hardness with heating for substantially 150 hours were substantially the same in both the tap water and the aqueous solution. Conversely, the simulated water with which no $K_3PO_4$ was added reached a substantially equilibrium state by heating for substantially 150 hours. Normally, added $K_3PO_4$ is ionized, and $PO_4^{3-}$ immediately changes ($HPO_4^{2-} \rightarrow PO_4^{3-} + H^+$ (dissociation constant, Ka = $10^{-12.67}$)) to $HPO_4^{2-}$ at 7.2 < pH < 12.7. That is, $K_3PO_4$ that was added to the simulated water changed to $HPO_4^{2-}$, and a $CaCO_3$ deposition reaction was suppressed by the same effect as that of $HPO_4^{2-}$ contained in the tap water.

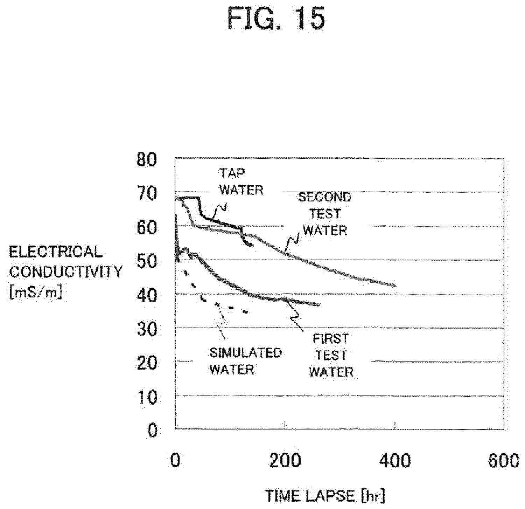

FIG. 15 is a diagram illustrating a time change in electrical conductivity of sample water during heating according to the first example. The deposition reaction of $CaCO_3$ causes the ion concentration in a solution to be decreased, and thus the electrical conductivity of each sample water decreases. The electrical conductivity of the tap water gently transitioned within a range between 68 mS/m and 69 mS/m up to substantially 50 hours after the start of heating, thereafter, the electrical conductivity started to decrease, and it took equal to or longer than 400 hours to reach a steady state (the transition to reach the steady state is not shown).

As illustrated in FIG. 15, in the case of the sample waters other than the tap water, the greater the amount of $K_3PO_4$ added to the simulated water was, the more gently the electrical conductivity decreased. The simulated water with which no $K_3PO_4$ was added, and the aqueous solution (first test water) that was the simulated water added with $K_3PO_4$ of 1 mg/L reached the steady state substantially 150 hours later, and substantially 250 hours later, respectively. In the case of the aqueous solution (second test water) that was the simulated water added with $K_3PO_4$, of 5 mg/L, the electrical conductivity gently transitioned within a range between 56 and 67 mS/m up to substantially 150 hours after the start of heating, thereafter the electrical conductivity started to decrease, and it took equal to or longer than 400 hours to reach the steady state.

However, in the above-shown results, because the absolute values of respective initial electrical conductivities were different, a comparison of the rate of $CaCO_3$ deposition reaction cannot be performed with just the absolute values. Thus, a normalized "rate of change of the electrical conductivity" based on the initial value of the electrical conductivity in each test and a steady-state value thereof was applied, and a time $t_1$ that was needed to reach a reaching rate of 20 % to the equilibrium state was compared.

As a result, the simulated water with which no $K_3PO_4$ was added took $t_1$ = 1.6 hours, the aqueous solution (first test water) that was the simulated water added with $K_3PO_4$ of 1 mg/L took $t_1$ = 5 hours, and the aqueous solution (second test water) that was the simulated water added with $K_3PO_4$ of 5 mg/L took $t_1$ = 28 hours. Conversely, the tap water took $t_1$ = 61 hours. Next, the addition amount of $K_3PO_4$ was further increased, and the rate of change of the electrical conductivity was measured likewise in the aqueous solution having the $HPO_4^{2-}$ concentration of equal to or greater than 3.0 mg/L. In addition, the time $t_1$ that was needed to reach the reaching rate of 20 % to the equilibrium state was evaluated, and in the case of the aqueous solution having the $HPO_4^{2-}$ concentration of equal to or greater than 3.0 mg/L, it took $t_1$ = 60 hours that was constant (not shown).

The deposition rate R of $CaCO_3$ is proportional to the rate of change in the electrical conductivity per unit time, thus inversely proportional to the time $t_1$ needing to reach the reaching rate of 20 % to the equilibrium state. Therefore, when the deposition rate of the simulated water with which no $K_3PO_4$ was added is R = 100, in the case of the aqueous solution (first test water) ($HPO_4^{2-}$ concentration of 0.4 mg/L) that was the simulated water added with $K_3PO_4$, of 1 mg/L, the deposition rate was R = 32, and in the case of the aqueous solution (second test water) ($HPO_4^{2-}$ concentration 2.2 mg/L) that was the simulated water added with $K_3PO_4$, of 5 mg/L, the deposition rate was R = 5.7. In the case of the tap water ($HPO_4^{2-}$ concentration 3.2 mg/L), the deposition rate was R = 2.6.

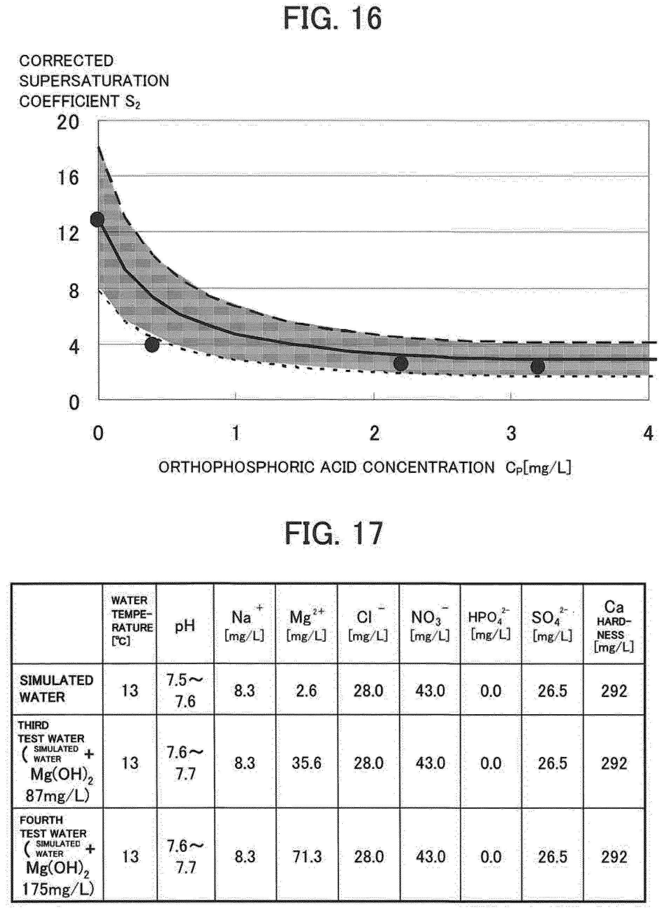

FIG. 16 is a diagram illustrating a value of a modified supersaturation coefficient to an orthophosphoric acid concentration according to the first example. A hatched area in FIG. 16 indicates a range for the modified supersaturation coefficient expressed by the aforementioned formula 5 and formula 6. A solid line indicates a representative value that passes through the center of the range.

Based on the results of the first example as shown above, it becomes clear that although the Ca hardness (calcium ion concentration), the alkali level corresponding to the amount of carbonate ions, the pH in the aqueous solution, and the water temperature are the same, the $CaCO_3$ deposition rate R remarkably changes in accordance with the orthophosphoric ion concentration ($HPO_4^{2-}$ concentration) in the aqueous solution.

Second Example

In a second example, like the aforementioned first example, a test result by using the scale deposition test device 600, performing a nitrogen gas aeration in advance, and maintaining various sample waters with the pH being adjusted from 8.4 to 8.7 at 65 °C for equal to or longer than several hundred hours is shown. The various sample waters used herein were, the simulated water like the aforementioned first example, an aqueous solution (third test water) that was the simulated water added with $Mg(OH)_2$ of 87 mg/L, and an aqueous solution (fourth test water) that was the simulated water added with $Mg(OH)_2$ of 175 mg/L.

FIG. 17 is a diagram illustrating a water quality analysis result before heating according to the second example. As illustrated in FIG. 17, the aqueous solution (third test water) that was the simulated water added with $Mg(OH)_2$ of 87 mg/L had an $Mg^{2+}$ concentration of 35.6 mg/L ($1.5 \times 10^{-3}$ mol/L), and the aqueous solution (fourth test water) that was the simulated water added with $Mg(OH)_2$ of 175 mg/L had the $Mg^{2+}$ concentration of 71.3 mg/L ($3.0 \times 10^{-3}$ mol/L). Note that since the simulated water contained substantially $3.0 \times 10^{-3}$ mol/L of $Ca^{2+}$, the molar concentration ratio of $Mg^{2+}/Ca^{2+}$ was 0.5 in the case of the third test water, and was 1.0 in the case of the fourth test water.

Like the aforementioned first example, when heating was conducted, the pH of each sample water gradually decreased through the heating for several hundred hours, and reached to 7.6 to 8.2 (not shown). As discussed above, this is presumably because $CO_3^{2-}$ was consumed along with the deposition of calcium carbonate, causing a shift of the equilibrium to the right for the dissociation reaction ($HCO_3^- \leftrightarrow H^+ + CO_3^{2-}$) of $HCO_3^-$, and thus $H^+$ was produced.

In each sample water, the supersaturation coefficient (a ratio of the amount of ions in a solution to the amount of ions in a saturation state) to calcium carbonate before heating was 112 to 165 times. However, after heating, the supersaturation coefficient was decreased to 1.5 to 2.5 times, and reached a substantially equilibrium state. Note that when X-ray diffraction of solid particles deposited in the aqueous solution was evaluated, calcium carbonate ($CaCO_3$) was contained but no magnesium carbonate ($MgCO_3$) was contained.

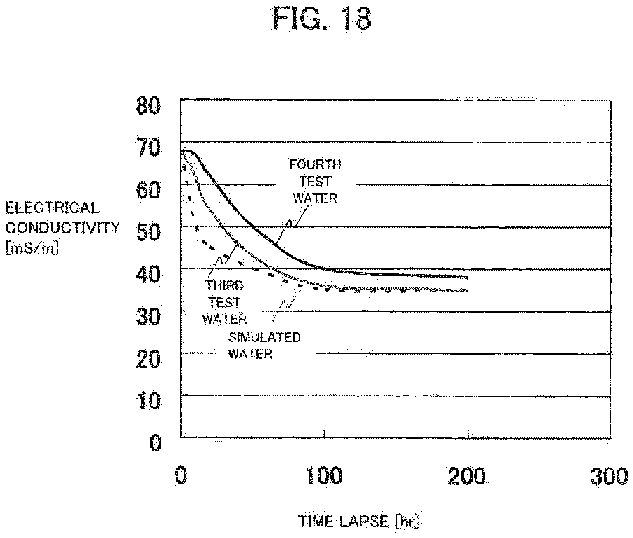

FIG. 18 is a diagram illustrating a time change in electrical conductivity of sample water during heating according to the second example. The deposition reaction of $CaCO_3$ causes the ion concentration in a solution to be decreased, and thus the electrical conductivity of the solution decreases. The greater the amount of magnesium hydroxide added to the simulated water becomes, the more gently the electrical conductivity of the simulated water decreases, and, as illustrated in FIG. 18, the simulated water with no magnesium hydroxide, and the third test water having the $Mg^{2+}$ concentration of 35.6 mg/L ($1.5 \times 10^{-3}$ mol/L) reached the equilibrium state after substantially 150 hours, and after substantially 180 hours, respectively. In the case of the fourth test water having the $Mg^{2+}$ concentration of 71.3 mg/L ($3.0 \times 10^{-3}$ mol/L), the electrical conductivity gently transitioned within a range between 60 and 67 mS/m up to substantially 30 to 40 hours after the start of heating, thereafter, the electrical conductivity started to decrease, and it took equal to or longer than 200 hours to reach the steady state.

Based on an initial value of the electrical conductivity in each test and a steady-state value thereof, a normalized "rate of change of the electrical conductivity" was applied, and the time $t_1$ that was needed to reach the reaching rate of 20% to the equilibrium state was compared. As a result, the simulated water with which no magnesium hydroxide was added took $t_1 = 1.6$ hours, the third test water having the $Mg^{2+}$ concentration of 35.6 mg/L ($1.5 \times 10^{-3}$ mol/L) took $t_1 = 4$ hours, and the fourth test water having the $Mg^{2+}$ concentration of 71.3 mg/L ($3.0 \times 10^{-3}$ mol/L) took $t_1 = 10$ hours.

The deposition rate R of $CaCO_3$ is proportional to the rate of change in the electrical conductivity per unit time, thus inversely proportional to the time $t_1$ needing to reach the reaching rate of 20% to the equilibrium state. Therefore, when the deposition rate of the simulated water with which none of $K_3PO_4$ and magnesium hydroxide was added is R = 100, in the case of the third test water having the $Mg^{2+}$ concentration of 35.6 mg/L ($1.5 \times 10^{-3}$ mol/L), the deposition rate was R = 40, and in the case of the fourth test water having the $Mg^{2+}$ concentration of 71.3 mg/L ($3.0 \times 10^{-3}$ mol/L), the deposition rate was R = 16.

That is, when the molar concentration ratio of $Mg^{2+}/Ca^{2+}$ was 0.5, the deposition rate was R = 40, and when the molar concentration ratio of $Mg^{2+}/Ca^{2+}$ was 1.0, the deposition rate was R = 16. When the same test was conducted using the aqueous solution having the $Ca^{2+}$ concentration within a range from 1 to 1000 mg/L, it becomes clear that, regardless of the $Ca^{2+}$ concentration, the deposition rate was R = 40 when the molar concentration ratio of $Mg^{2+}/Ca^{2+}$ was 0.5, and the deposition rate was R = 16 when the molar concentration ratio of $Mg^{2+}/Ca^{2+}$ was 1.0 (not shown). Note that, like the aforementioned first example, the aforementioned formula 7 was obtained based on a relationship of the modified supersaturation coefficient with the molar concentration ratio of $Mg^{2+}/Ca^{2+}$ (not shown).

According to the results of the second example as shown above, it becomes clear that although the Ca hardness (calcium ion concentration), the alkali level corresponding to the amount of carbonate ions, the pH in the aqueous solution, and the water temperature are the same, magnesium ion in the solution remarkably affects the deposition rate R of $CaCO_3$. When the results of the first example are taken into consideration, it becomes clear that, in addition to orthophosphoric ion and magnesium ion, ions like silicate ions that react with calcium ion, and that produce salt with low solubility also affect the deposition rate R of $CaCO_3$.

List of Reference Signs

| | |
|---|---|
| 1 | Hot-water feeder system |
| 2 | Compressor |
| 3 | First coolant piping |
| 4 | Coolant/water heat exchanger |
| 5 | Hot water tank |
| 6 | Pump |
| 7 | First water piping |
| 8 | Second water piping |
| 9 | Second coolant piping |
| 10 | Expansion valve |
| 11 | Third coolant piping |
| 12 | Evaporator |
| 13 | Fan |
| 14 | Fourth coolant piping |
| 15 | First water supply piping |
| 16 | Second water supply piping |
| 17 | Water quality controller |
| 18 | First flow-rate control valve |
| 19 | Second flow-rate control valve |
| 20 | Conductivity meter |
| 21 | Water quality checking faucet |
| 22 | Bypass circuit piping |
| 23 | Automatic open/close valve |
| 24 | Scale particle catcher |
| 25 | Third flow-rate control valve |
| 26 | Fourth flow-rate control valve |
| 100 | Water quality checking device |
| 101 | Measurer |
| 102 | Calculator |
| 103 | Corrector |
| 104 | Determiner |
| 201,301,401 | Water quality checking controller |
| 202, 402 | Water quality measurer |
| 303 | Water temperature sensor |
| 600 | Scale deposition test device |
| 601 | Heat-resistant glass vessel |
| 602 | Electrical-conductivity electrode |
| 603 | Silicone rubber |
| 604 | Magnetic stirrer |
| 605 | Thermostatic bath |

**Claims**

**1.**   A method for checking a water quality,

the method **characterized by** comprising:

- a first process of measuring, in a solution subjected to water quality checking, respective values of a calcium ion concentration, a carbonate ion concentration, a hydrogen ion concentration, a water temperature, and a concentration of dissolved ion that reacts with calcium ion, and that produces salt with low solubility;
- a second process of calculating, based on the values of the calcium ion concentration, the carbonate ion concentration, the hydrogen ion concentration and the water temperature that are measured in the first process, a supersaturation coefficient of calcium carbonate in the solution;
- a third process of calculating, by applying the concentration value of the dissolved ion that reacts with the calcium ion and that produces the salt with low solubility, the concentration value being measured through the first process, a modified supersaturation coefficient that is corrected from the supersaturation coefficient; and
- a fourth process of determining the water quality based on the modified supersaturation coefficient.

2. The water quality checking method according to Claim 1,
**characterized in that**:

the dissolved ion that reacts with the calcium ion and that produces the salt with low solubility is orthophosphoric ion, and when a value of orthophosphoric ion concentration is $C_P$ (mg/L), in the third process, from the supersaturation coefficient $S_1$:

when $C_P \geq 3.0$, as to the modified supersaturation coefficient $S_2$, a value within the following range is applied:

[Formula 1]

$$0.136S_1 \leq S_2 \leq 0.318S_1, \text{ and}$$

and when $C_P < 3.0$, as to the modified supersaturation coefficient $S_2$, a value within the following range is applied.

[Formula 2]

$$\frac{0.6S_1}{(-0.29C_p^2 + 2.0C_p + 1)} \leq S_2 \leq \frac{1.4S_1}{(-0.29C_p^2 + 2.0C_p + 1)}$$

3. The water quality checking method according to Claim 1,
**characterized in that**:
the dissolved ion that reacts with the calcium ion and that produces the salt with low solubility is magnesium ion, and when a value of magnesium ion concentration is $C_M$ (mol/L), and a value of the calcium ion concentration is $C_C$ (mol/L), in the third process, from the supersaturation coefficient $S_1$, as to the modified supersaturation coefficient $S_2$, a value within the following range is applied.

[Formula 3]

$$\frac{0.6S_1}{\{1 + 0.2(C_M / C_C)\}} \leq S_2 \leq \frac{1.4S_1}{\{1 + 0.2(C_M / C_C)\}}$$

4. The water quality checking method according to any one of Claims 1 to 3, **characterized in that** in the fourth process, the water quality is determined based on a determination on whether or not the modified supersaturation coefficient is equal to or greater than a threshold.

5. The water quality checking method according to any one of Claims 1 to 3, **characterized in that** in the fourth process:

an amount of scale build-up within an arbitrary use period is calculated based on the modified supersaturation coefficient and on a basis of, in predetermined water quality containing no dissolved ion other than carbonate ion that reacts with the calcium ion and that produces the salt with low solubility, an induction time period until scales are deposited and a growth speed of the scales; and
the water quality is determined based on a determination on whether or not the scale build-up amount is equal to or greater than a threshold.

6. The water quality checking method according to any one of Claims 1 to 3, **characterized in that** in the fourth process, a time until an amount of scale build-up based on the modified supersaturation coefficient reaches an allowable scale build-up amount is calculated on a basis of, in predetermined water quality containing no dissolved ion other than carbonate ion that reacts with the calcium ion and that the produces the salt with low solubility, an induction time period until scales are deposited and a growth speed of the scales, and the water quality is determined based on an indicator that is the calculated time.

7. A water quality checking device (100)
**characterized by** comprising:

- a measurer (101) that is configured to measure, in a solution subjected to water quality checking, respective values of a calcium ion concentration, a carbonate ion concentration, a hydrogen ion concentration, a water temperature, and a concentration of dissolved ion that reacts with calcium ion, and that produces salt with low solubility;
- a calculator (102) that is configured to calculate, based on the values of the calcium ion concentration, the carbonate ion concentration, the hydrogen ion concentration and the water temperature that are measured by the measurer (101), a supersaturation coefficient of calcium carbonate in the solution;
- a corrector (103) that is configured to calculate, by applying the concentration value of the dissolved ion that reacts with the calcium ion and that produces the salt with low solubility, the concentration value being measured by the measurer (101), a modified supersaturation coefficient that is corrected from the supersaturation coefficient; and
- a determiner (104) that is configured to determine the water quality based on the modified supersaturation coefficient.

8. The water quality checking device (100) according to Claim 7,
**characterized in that**:
the dissolved ion that reacts with the calcium ion and that produces the salt with low solubility is orthophosphoric ion, and when a value of orthophosphoric ion concentration is $C_P$ (mg/L), the corrector (103) is configured to calculate, from the supersaturation coefficient $S_1$:
a value given within the following range as to the modified supersaturation coefficient
$S_2$ when $C_P \geq 3.0$:

[Formula 4]
$$0.136S_1 \leq S_2 \leq 0.318S_1,$$

and
a value given within the following range as to the modified supersaturation coefficient $S_2$ when $C_P < 3.0$.

[Formula 5]
$$\frac{0.6S_1}{(-0.29C_p^2 + 2.0C_p + 1)} \leq S_2 \leq \frac{1.4S_1}{(-0.29C_p^2 + 2.0C_p + 1)}$$

9. The water quality checking device (100) according to Claim 7,
**characterized in that**:
the dissolved ion that reacts with the calcium ion and that the produces the salt with low solubility is magnesium ion, and when a value of magnesium ion concentration is $C_M$ (mol/L), and a value of the calcium ion concentration is $C_C$ (mol/L), the corrector (103) is configured to calculate, from the supersaturation coefficient $S_1$, as to the modified

supersaturation coefficient $S_2$, a value given within the following range is applied.

[Formula 6]

$$\frac{0.6S_1}{\{1+0.2(C_M/C_C)\}} \leq S_2 \leq \frac{1.4S_1}{\{1+0.2(C_M/C_C)\}}$$

**10.** The water quality checking device (100) according to any one of Claims 7 to 9, **characterized in that** the determiner (104) is configured to determine the water quality based on a determination on whether or not the modified supersaturation coefficient is equal to or greater than a threshold.

**11.** The water quality checking device (100) according to any one of Claims 7 to 9, **characterized in that** the determiner (104):

is configured to calculate an amount of scale build-up within an arbitrary use period based on the modified supersaturation coefficient and on a basis of, in predetermined water quality containing no dissolved ion other than carbonate ion that reacts with the calcium ion and that produces the salt with low solubility, an induction time period until scales are deposited and a growth speed of the scales;
and is configured to determine the water quality based on a determination on whether or not the scale build-up amount is equal to or greater than a threshold.

**12.** The water quality checking device (100) according to any one of Claims 7 to 9, **characterized in that** the determiner (104) is configured to calculate a time until an amount of scale build-up based on the modified supersaturation coefficient reaches an allowable scale build-up amount on a basis of, in predetermined water quality containing no dissolved ion other than carbonate ion that reacts with the calcium ion and that produces the salt with low solubility, an induction time period until scales are deposited and a growth speed of the scales, and determine the water quality based on an indicator that is the calculated time.

**13.** A hot-water feeder system (1) **characterized by** comprising the water quality checking device (100) according to any one of Claims 7 to 12, and checking a water quality of water that flows through a heat exchanger (4).

**14.** A hot-water feeder system (1) **characterized by** comprising:

the water quality checking device (100) according to Claim 10 or 11:

- a controller that is configured to improve water quality of water flowing through a heat exchanger (4) so as to decrease the modified supersaturation coefficient or the scale build-up amount when the determiner (104) determines that the modified supersaturation coefficient or the scale build-up amount is equal to or greater than the threshold,

**characterized in that** the system checks the water quality of the water flowing through the heat exchanger (4).

**15.** The hot-water feeder system (1) according to Claim 14,
**characterized in that** the controller comprises:

- a water quality controller (17) filled with a substance that is configured to provide sustained-release of orthophosphoric ion or magnesium ion; and
- a flow-rate control valve (18) that is configured to make adjustment to cause water supplied to a hot water tank (5) to flow through the water quality controller (17).

**Patentansprüche**

**1.** Verfahren zum Überprüfen der Wasserqualität, wobei das Verfahren
**dadurch gekennzeichnet ist, dass** es folgende Vorgänge aufweist:

- einen ersten Vorgang zum Messen, und zwar in einer Lösung, die einer Wasserqualitätsüberprüfung unterzogen wird, der jeweiligen Werte einer Calciumionenkonzentration, einer Carbonationenkonzentration, einer Wasserstoffionenkonzentration, einer Wassertemperatur und einer Konzentration gelöster Ionen, die mit den Calciumionen reagieren und die das Salz mit geringer Löslichkeit erzeugen;

- einen zweiten Vorgang zum Berechnen, und zwar basierend auf den Werten der Calciumionenkonzentration, der Carbonationenkonzentration, der Wasserstoffionenkonzentration und der Wassertemperatur, die in dem ersten Vorgang gemessen werden, eines Übersättigungskoeffizienten von Calciumcarbonat in der Lösung;

- einen dritten Vorgang zum Berechnen eines modifizierten Übersättigungskoeffizienten, der aus dem Übersättigungskoeffizienten korrigiert wird, und zwar durch Anwenden des Konzentrationswertes der gelösten Ionen, die mit den Calciumionen reagieren und die das Salz mit geringer Löslichkeit erzeugen, wobei der Konzentrationswert mittels des ersten Vorgangs gemessen wird; und

- einen vierten Vorgang zum Bestimmen der Wasserqualität basierend auf dem modifizierten Übersättigungskoeffizienten.

2. Verfahren zum Überprüfen der Wasserqualität nach Anspruch 1,
**dadurch gekennzeichnet, dass**
die gelösten Ionen, die mit den Calciumionen reagieren und die das Salz mit geringer Löslichkeit erzeugen, orthophosphorisches Ionen sind, und wenn ein Wert der orthophosphorischen Ionenkonzentration CP (mg/L) ist, in dem dritten Vorgang ausgehend von dem Übersättigungskoeffizienten $S_1$ gilt:

wenn $C_P \geq 3{,}0$, bezüglich des modifizierten Übersättigungskoeffizienten $S_2$, ein Wert innerhalb des folgenden Bereichs angewendet wird:

$$[\text{Formel 1}]$$
$$0{,}136\,S_1 \leq S_2 \leq 0{,}318\,S_1, \text{ und}$$

wenn $C_P < 3{,}0$, bezüglich des modifizierten Übersättigungskoeffizienten $S_2$, ein Wert innerhalb des folgenden Bereichs angewendet wird:

$$[\text{Formel 2}]$$
$$\frac{0.6S_1}{(-0.29C_p^2 + 2.0C_p + 1)} \leq S_2 \leq \frac{1.4S_1}{(-0.29C_p^2 + 2.0C_p + 1)}$$

3. Verfahren zum Überprüfen der Wasserqualität nach Anspruch 1,
**dadurch gekennzeichnet, dass**
die gelösten Ionen, die mit dem Calciumionen reagieren und die das Salz mit geringer Löslichkeit erzeugen, Magnesiumionen sind, und wenn ein Wert der Magnesiumionenkonzentration $C_M$ (mol/L) und ein Wert der Calciumionenkonzentration $C_C$ (mol/L) ist, in dem dritten Vorgang ausgehend von dem Übersättigungskoeffizienten $S_1$ bezüglich des modifizierten Übersättigungskoeffizienten $S_2$ ein Wert innerhalb des folgenden Bereichs angewendet wird:

$$[\text{Formel 3}]$$
$$\frac{0.6S_1}{\{1 + 0.2(C_M / C_C)\}} \leq S_2 \leq \frac{1.4S_1}{\{1 + 0.2(C_M / C_C)\}}$$

4. Verfahren zum Überprüfen der Wasserqualität nach einem der Ansprüche 1 bis 3,
**dadurch gekennzeichnet, dass**
in dem vierten Vorgang die Wasserqualität basierend auf einer Bestimmung bestimmt wird, ob oder ob nicht der modifizierte Übersättigungskoeffizient gleich wie oder größer als ein Schwellenwert ist.

**5.** Verfahren zum Überprüfen der Wasserqualität nach einem der Ansprüche 1 bis 3, **dadurch gekennzeichnet, dass** in dem vierten Vorgang:

eine Menge an Kesselsteinbildung innerhalb einer beliebigen Verwendungsperiode berechnet wird, und zwar basierend auf dem modifizierten Übersättigungskoeffizienten und basierend auf einer Induktionszeitperiode bis Kesselstein abgelagert wird und einer Wachstumsgeschwindigkeit der Kesselsteine, und zwar in einer vorbestimmten Wasserqualität, die keine anderen gelösten Ionen als Carbonationen enthält, die mit dem Calcium reagieren und das Salz mit geringer Löslichkeit erzeugen; und
wobei die Wasserqualität basierend auf einer Bestimmung bestimmt wird, ob oder ob nicht die Menge an Kesselsteinbildung gleich wie oder größer als ein Schwellenwert ist.

**6.** Verfahren zum Überprüfen der Wasserqualität nach einem der Ansprüche 1 bis 3, **dadurch gekennzeichnet, dass**
in dem vierten Vorgang eine Zeitspanne, bis eine auf dem modifizierten Übersättigungskoeffizienten basierende Menge an Kesselsteinbildung eine zulässige Menge an Kesselsteinbildung erreicht, basierend auf einer Induktionszeitperiode bis sich Kesselstein ablagert und einer Wachstumsgeschwindigkeit der Kesselsteine berechnet wird, und zwar in einer vorbestimmter Wasserqualität, die keine anderen gelösten Ionen außer Carbonationen enthält, die mit den Calciumionen reagieren und die das Salz mit geringer Löslichkeit erzeugen, und
wobei die Wasserqualität basierend auf einem Indikator bestimmt wird, der die berechnete Zeit darstellt.

**7.** Vorrichtung (100) zum Überprüfen der Wasserqualität **dadurch gekennzeichnet, dass**
die Vorrichtung Folgendes aufweist:

- eine Messvorrichtung (101), die dazu ausgebildet ist, in einer Lösung, die einer Wasserqualitätsüberprüfung unterzogen wird, jeweilige Werte einer Calciumionenkonzentration, einer Carbonationenkonzentration, einer Wasserstoffionenkonzentration, einer Wassertemperatur, und einer Konzentration gelöster Ionen zu messen, die mit Calciumionen reagieren und die ein Salz mit geringer Löslichkeit erzeugen;
- eine Berechnungseinrichtung (102), die dazu ausgebildet ist, basierend auf den Werten der Calciumionenkonzentration, der Carbonationenkonzentration, der Wasserstoffionenkonzentration und der Wassertemperatur, die von der Messvorrichtung (101) gemessen werden, einen Übersättigungskoeffizienten von Calciumcarbonat in der Lösung zu berechnen;
- eine Korrektureinrichtung (103), die dazu ausgebildet ist, mittels Anwendens des Konzentrationswertes der gelösten Ionen, die mit dem Calciumionen reagieren und die das Salz mit geringer Löslichkeit erzeugen, einen modifizierten Übersättigungskoeffizienten zu berechnen, der aus dem Übersättigungskoeffizienten korrigiert wird, wobei der Konzentrationswert mittels der Messvorrichtung (101) gemessen wird, und
- eine Bestimmungseinrichtung (104), die dazu ausgebildet ist, die Wasserqualität basierend auf dem modifizierten Übersättigungskoeffizienten zu bestimmen.

**8.** Vorrichtung (100) zum Überprüfen der Wasserqualität nach Anspruch 7, **dadurch gekennzeichnet, dass**
die gelösten Ionen, die mit dem Calciumionen reagieren und die das Salz mit geringer Löslichkeit erzeugen, orthophosphorische Ionen sind, und wenn ein Wert der orthophosphorischen Ionenkonzentration $C_P$ (mg/L) ist, die Korrektureinrichtung (103) dazu ausgebildet ist, aus dem Übersättigungskoeffizienten $S_1$ Folgendes zu berechnen:
einen Wert für den modifizierten Übersättigungskoeffizienten $S_2$, wenn $C_P \geq 3{,}0$ ist, und zwar innerhalb des folgenden Bereichs:

[Formel 4]

$$0{,}136\,S_1 \leq S_2 \leq 0{,}318\,S_1,$$

und
wenn $C_P < 3{,}0$, einen Wert für den modifizierten Übersättigungskoeffizienten $S_2$ innerhalb des folgenden Bereichs:

[Formel 5]

$$\frac{0.6S_1}{(-0.29C_p^2 + 2.0C_p + 1)} \leq S_2 \leq \frac{1.4S_1}{(-0.29C_p^2 + 2.0C_p + 1)}$$

**9.** Vorrichtung (100) zum Überprüfen der Wasserqualität nach Anspruch 7, **dadurch gekennzeichnet, dass** die gelösten Ionen, die mit dem Calciumionen reagieren und die das Salz mit geringer Löslichkeit erzeugen, Magnesiumionen sind, und wenn ein Wert der Magnesiumionenkonzentration $C_M$ (mol/L) ist und ein Wert der Calciumionenkonzentration $C_C$(mol/L) ist, die Korrektureinrichtung (103) dazu ausgebildet ist, aus dem Übersättigungskoeffizienten $S_1$ für den modifizierten Übersättigungskoeffizienten $S_2$ einen Wert zu berechnen, der innerhalb des folgenden Bereichs liegt:

[Formel 6]

$$\frac{0.6S_1}{\{1 + 0.2(C_M / C_C)\}} \leq S_2 \leq \frac{1.4S_1}{\{1 + 0.2(C_M / C_C)\}}$$

**10.** Vorrichtung (100) zum Überprüfen der Wasserqualität nach einem der Ansprüche 7 bis 9, **dadurch gekennzeichnet, dass** die Bestimmungseinrichtung (104) dazu ausgebildet ist, die Wasserqualität basierend auf einer Bestimmung zu bestimmen, ob oder ob nicht der modifizierte Übersättigungskoeffizient gleich wie oder größer als ein Schwellenwert ist.

**11.** Vorrichtung (100) zum Überprüfen der Wasserqualität nach einem der Ansprüche 7 bis 9, **dadurch gekennzeichnet, dass** die Bestimmungseinrichtung (104):

dazu ausgebildet ist, eine Menge an Kesselsteinbildung innerhalb einer beliebigen Verwendungsperiode zu berechnen, und zwar basierend auf dem modifizierten Übersättigungskoeffizienten und basierend auf einer Induktionszeitsperiode bis sich die Kesselsteine ablagern und einer Wachstumsgeschwindigkeit der Kesselsteine, und zwar in einer vorbestimmten Wasserqualität, die keine gelösten Ionen außer Carbonationen enthält, die mit den Calciumionen reagieren und die das Salz mit geringer Löslichkeit erzeugt;
und dazu ausgebildet ist, die Wasserqualität basierend auf einer Bestimmung zu bestimmen, ob oder ob nicht die Menge an Kesselsteinbildung gleich wie oder größer als ein Schwellenwert ist.

**12.** Vorrichtung (100) zum Überprüfen der Wasserqualität nach einem der Ansprüche 7 bis 9, **dadurch gekennzeichnet, dass** die Bestimmungseinrichtung (104) dazu ausgebildet ist, eine Zeitspanne zu berechnen, bis eine Menge an Kesselsteinbildung, und zwar basierend auf dem modifizierten Übersättigungskoeffizienten, eine zulässige Menge an Kesselsteinbildung erreicht, und zwar basierend auf einer Induktionszeitperiode bis Kesselsteine abgelagert werden und einer Wachstumsgeschwindigkeit der Kesselsteine, und zwar in einer vorbestimmten Wasserqualität, die keine gelösten Ionen außer Carbonationen enthält, die mit den Calciumionen reagieren und die das Salz mit geringer Löslichkeit erzeugen, und die Wasserqualität basierend auf einem Indikator zu bestimmen, der die berechnete Zeit darstellt.

**13.** Warmwasser-Zufuhrsystem (1), **dadurch gekennzeichnet, dass** es die Vorrichtung (100) zum Überprüfen der Wasserqualität gemäß einem der Ansprüche 7 bis 12 aufweist und eine Wasserqualität von Wasser überprüft, das durch einen Wärmetauscher (4) strömt.

**14.** Warmwasser-Zufuhrsystem (1), **dadurch gekennzeichnet, dass** es Folgendes aufweist:

- die Vorrichtung (100) zum Überprüfen der Wasserqualität nach Anspruch 10 oder 11:
- eine Steuerung, die dazu ausgebildet ist, die Wasserqualität des durch einen Wärmetauscher (4) fließenden Wassers zu verbessern, um den modifizierten Übersättigungskoeffizienten oder die Menge an Kesselsteinbildung zu verringern, wenn die Bestimmungseinrichtung (104) bestimmt, dass der modifizierte Übersättigungskoeffizient oder die Menge an Kesselsteinbildung gleich wie oder größer als der Schwellenwert ist,

**dadurch gekennzeichnet, dass**
das System die Wasserqualität des durch den Wärmetauscher (4) fließenden Wassers überprüft.

**15.** Warmwasser-Zufuhrsystem (1) gemäß Anspruch 14,
**dadurch gekennzeichnet, dass** die Steuerung Folgendes aufweist:

- eine Wasserqualitäts-Steuerung (17), die mit einer Substanz gefüllt ist, die dazu ausgebildet ist, eine anhaltende Freisetzung von orthophosphorischen Ionen oder Magnesiumionen bereitzustellen; und
- ein Flussraten-Steuerventil (18), das dazu ausgebildet ist, eine Einstellung vorzunehmen, um zu veranlassen, dass das dem Warmwasserbehälter (5) zugeführte Wasser durch die Wasserqualitäts-Steuerung (17) fließt.

## Revendications

**1.** Procédé pour vérifier la qualité de l'eau,
le procédé étant **caractérisé en ce qu'**il comprend:

- un premier processus consistant à mesurer, dans une solution soumise à une vérification de la qualité de l'eau, des valeurs respectives d'une concentration en ions calcium, une concentration en ions carbonate, une concentration en ions hydrogène, une température de l'eau, et une concentration des ions dissous qui réagissent avec les ions calcium, et qui produit du sel avec une faible solubilité;
- un second processus consistant à calculer, sur la base des valeurs de la concentration en ions calcium, de la concentration en ions carbonate, de la concentration en ions hydrogène et de la température de l'eau qui sont mesurées dans le premier processus, un coefficient de sursaturation de carbonate de calcium dans la solution;
- un troisième processus consistant à calculer, en appliquant la valeur de concentration des ions dissous qui réagissent avec les ions calcium et qui produisent le sel à faible solubilité, la valeur de concentration étant mesurée par le premier processus, un coefficient de sursaturation modifié qui est corrigé par rapport au coefficient de sursaturation; et
- un quatrième processus consistant à déterminer la qualité de l'eau sur la base du coefficient de sursaturation modifié.

**2.** Procédé de vérification de la qualité de l'eau selon la revendication 1,
**caractérisé en ce que**:
les ions dissous qui réagissent avec les ions calcium et qui produisent le sel à faible solubilité sont des ions orthophosphoriques et, quand une valeur de concentration des ions orthophosphoriques est Cp (mg/L), dans le troisième processus, à partir du coefficient de sursaturation $S_1$:
quand $C_P \geq 3{,}0$, à titre de coefficient de sursaturation modifié $S_2$, on applique une valeur à l'intérieur de la plage suivante:

$$[\text{formule 1}]$$

$$0{,}136\,S_1 \leq S_2 \leq 0{,}318\,S_1,$$

et
quand $C_P < 3{,}0$, à titre de coefficient de sursaturation modifié $S_2$, on applique une valeur à l'intérieur de la plage suivante :

[formule 2]

$$\frac{0.6S_1}{(-0.29C_p^2 + 2.0C_p + 1)} \leq S_2 \leq \frac{1.4S_1}{(-0.29C_p^2 + 2.0C_p + 1)}$$

**3.** Procédé de vérification de la qualité de l'eau selon la revendication 1,
**caractérisé en ce que**:
les ions dissous qui réagissent avec les ions calcium et qui produisent le sel à faible solubilité sont des ions magnésium et, quand une valeur de la concentration en ions magnésium est $C_M$ (mole/L) et une valeur de la concentration en ions calcium est $C_C$ (mole/L), dans le troisième processus, à partir du coefficient de sursaturation $S_1$, on applique à titre de coefficient de sursaturation modifié $S_2$ une valeur à l'intérieur de la plage suivante :

[formule 3]

$$\frac{0.6S_1}{\{1 + 0.2(C_M / C_C)\}} \leq S_2 \leq \frac{1.4S_1}{\{1 + 0.2(C_M / C_C)\}}$$

**4.** Procédé de vérification de la qualité de l'eau selon l'une quelconque des revendications 1 à 3,
**caractérisé en ce que** dans le quatrième processus, la qualité de l'eau est déterminée sur la base d'une détermination quant à savoir si le coefficient de sursaturation modifié est égal ou supérieur à un seuil ou non.

**5.** Procédé de vérification de la qualité de l'eau selon l'une quelconque des revendications 1 à 3,
**caractérisé en ce que** dans le quatrième processus : une quantité de déposition de tartre à l'intérieur d'une période d'utilisation arbitraire est calculée sur la base du coefficient de sursaturation modifié et sur la base, dans une eau de qualité prédéterminée ne contenant aucun ion dissous autre que les ions carbonates qui réagissent avec les ions calcium et qui produisent le sel à faible solubilité, d'une période temporelle d'induction jusqu'à ce que du tartre soit déposé et d'une vitesse de croissance du tartre ; et
la qualité de l'eau est déterminée sur la base d'une détermination quant à savoir si la quantité de déposition de tartre est égale ou supérieure à un seuil ou non.

**6.** Procédé de vérification de la qualité de l'eau selon l'une quelconque des revendications 1 à 3,
**caractérisé en ce que** dans le quatrième processus, un temps jusqu'à ce qu'une quantité de tartre déposé, basée sur le coefficient de sursaturation modifié, atteigne une quantité de déposition de tartre permissible, est calculé sur la base, dans une eau de qualité prédéterminée ne contenant aucun ion dissous autre que des ions carbonate qui réagissent avec les ions calcium et qui produisent le sel à faible solubilité, d'une période temporelle d'induction jusqu'à ce que du tartre soit déposé et d'une vitesse de croissance du tartre, et la qualité de l'eau est déterminée sur la base d'un indicateur qui est le temps calculé.

**7.** Dispositif de vérification de la qualité de l'eau (100),
**caractérisé en ce qu'**il comprend:

- un moyen de mesure (101) qui est configuré pour mesurer, dans une solution soumise à une vérification de la qualité de l'eau, des valeurs respectives d'une concentration en ions calcium, une concentration en ions carbonate, une concentration en ions hydrogène, une température de l'eau, et une concentration en ions dissous qui réagissent avec les ions calcium, et qui produisent un sel à faible solubilité;
- un moyen de calcul (102) qui est configuré pour calculer, sur la base des valeurs de la concentration en ions calcium, la concentration en ions carbonate, la concentration en ions hydrogène, et la température de l'eau qui sont mesurées par le moyen de mesure (101), un coefficient de sursaturation du carbonate de calcium dans la solution;
- un moyen de correction (103) qui est configuré pour calculer, en appliquant la valeur de concentration des ions dissous qui réagissent avec les ions calcium et qui produisent le sel à faible solubilité, la valeur de concentration étant mesurée par le moyen de mesure (101), un coefficient de sursaturation modifié qui est corrigé à partir du coefficient de sursaturation ; et
- un moyen de détermination (100) qui est configuré pour déterminer la qualité de l'eau sur la base du coefficient de sursaturation modifié.

8. Dispositif de vérification de la qualité de l'eau (100) selon la revendication 7, **caractérisé en ce que**:
les ions dissous qui réagissent avec les ions calcium et qui produisent le sel à faible solubilité sont des ions orthophosphoriques et, quand une valeur de concentration des ions orthophosphoriques est $C_P$ (mg/L), le correcteur (103) est configuré pour calculer, à partir du coefficient de sursaturation S1 :
une valeur donnée à l'intérieur de la plage suivante, à titre de coefficient de sursaturation modifié S2 lorsque $C_P \geq 3,0$:

$$[\text{formule 4}]$$

$$0,136\, S_1 \leq S_2 \leq 0,318\, S_1,$$

et
une valeur donnée à l'intérieur de la plage suivante à titre de coefficient de sursaturation modifié S2 lorsque $C_P < 3,0$

$$[\text{formule 5}]$$

$$\frac{0.6S_1}{(-0.29C_p'^2 + 2.0C_p + 1)} \leq S_2 \leq \frac{1.4S_1}{(-0.29C_p^2 + 2.0C_p + 1)}$$

9. Dispositif de vérification de la qualité de l'eau (100) selon la revendication 7, **caractérisé en ce que**:

- les ions dissous qui réagissent avec des ions calcium et qui produisent le sel à faible solubilité sont des ions magnésium et, lorsqu'une valeur de la concentration en ions magnésium est $C_M$ (mole/L) et une valeur de la concentration en ions calcium est $C_C$ (mole/L), le correcteur (103) et configuré pour calculer, à partir du coefficient de sursaturation $S_1$, à titre de coefficient de sursaturation modifié $S_2$, une valeur donnée à l'intérieur de la plage suivante:

$$[\text{formule 6}]$$

$$\frac{0.6S_1}{\{1 + 0.2(C_M / C_C)\}} \leq S_2 \leq \frac{1.4S_1}{\{1 + 0.2(C_M / C_C)\}}$$

10. Dispositif de vérification de la qualité de l'eau (100) selon l'une quelconque des revendications 7 à 9, **caractérisé en ce que** le moyen de détermination (100) est configuré pour déterminer la qualité de l'eau sur la base d'une détermination quant à savoir si le coefficient de sursaturation modifié est égal ou supérieur à un seuil ou non.

11. Dispositif de vérification de la qualité de l'eau (100) selon l'une quelconque des revendications 7 à 9, **caractérisé en ce que** le moyen de détermination (100):

est configuré pour calculer une quantité de tartre déposée à l'intérieur d'une période d'utilisation arbitraire sur la base du coefficient de sursaturation modifié et sur la base, dans une eau de qualité prédéterminée qui ne contient aucun ion dissous autre que les ions carbonate qui réagissent avec les ions calcium et qui produisent le sel à faible solubilité, d'une période temporelle d'induction jusqu'à ce que du tartre soit déposé et d'une vitesse de croissance du tartre; et
est configuré pour déterminer la qualité de l'eau sur la base d'une détermination quant à savoir si la quantité de tartre déposée est égale ou supérieure à un seuil ou non.

**12.** Dispositif de vérification de la qualité de l'eau (100) selon l'une quelconque des revendications 7 à 9, **caractérisé en ce que** le moyen de détermination (100) est configuré pour calculer un temps jusqu'à ce qu'une quantité de tartre déposée, basée sur le coefficient de sursaturation modifié, atteigne une quantité de tartre déposée permissible sur la base, dans une eau de qualité prédéterminée qui ne contient aucun ion dissous autre que les ions carbonate qui réagissent avec les ions calcium et qui produisent le sel à faible solubilité, d'une période temporelle d'induction jusqu'à ce que du tartre soit déposé et d'une vitesse de croissance du tartre, et détermine la qualité de l'eau sur la base d'un indicateur qui est le temps calculé.

**13.** Système de fourniture d'eau chaude (1) **caractérisé en ce qu'**il comprend le dispositif de vérification de la qualité de l'eau (100) selon l'une quelconque des revendications 7 à 12, et qui vérifie la qualité de l'eau qui s'écoule à travers un échangeur de chaleur (4).

**14.** Système de fourniture d'eau chaude (1) **caractérisé en ce qu'**il comprend:

- le dispositif de vérification de la qualité de l'eau (100) selon la revendication 10 ou 11;
- un contrôleur qui est configuré pour améliorer la qualité de l'eau qui s'écoule à travers un échangeur de chaleur (4) de manière à diminuer le coefficient de sursaturation modifié ou la quantité de tartre déposée quand le moyen de détermination (100) détermine que le coefficient de sursaturation modifié ou la quantité de tartre déposée est égal(e) ou supérieur(e) au seuil,

**caractérisé en ce que** le système vérifie la qualité de l'eau qui s'écoule à travers l'échangeur de chaleur (4).

**15.** Système de fourniture d'eau chaude (1) selon la revendication 14, **caractérisé en ce que** le contrôleur comprend:

- un contrôleur de qualité d'eau (17) rempli d'une substance qui est configurée pour assurer un dégagement soutenu d'ions orthophosphoriques ou d'ions magnésium ; et
- une valve de commande de débit (18) qui est configurée pour procéder à un ajustement afin d'amener l'eau fournie à un réservoir d'eau chaude (5) à s'écouler à travers le contrôleur de qualité d'eau (17).

# FIG. 1

# FIG. 2

METHOD FOR CHECKING WATER QUALITY

S11

MEASURE CALCIUM ION CONCENTRATION $[Ca^{2+}]$, CARBONATE ION CONCENTRATION $[CO_3{}^{2-}]$, HYDROGEN ION CONCENTRATION $[H^+]$, WATER TEMPERATURE T, AND CONCENTRATION OF DISSOLVED ION THAT REACTS WITH CALCIUM ION, AND PRODUCES SALT WITH LOW SOLUBILITY

S12

CALCULATE SUPERSATURATION COEFFICIENT $S_1$
$$S_1 = [Ca^{2+}] [CO_3{}^{2-}] f_D{}^2/K_{sp}$$
$*f_D$: ACTIVITY COEFFICIENT OF DIVALENT ION (CHANGES DEPENDING ON ELECTRICAL CONDUCTIVITY (FUNCTION OF $H^+$))
$*K_{sp}$: SOLUBILITY PRODUCT (FUNCTION OF T)

S13

CALCULATE CORRECTED SUPERSATURATION COEFFICIENT $S_2$ BASED ON SUPERSATURATION COEFFICIENT $S_1$ USING MEASURED VALUE OF CONCENTRATION OF DISSOLVED ION (ORTHOPHOSPHORIC ION OR MAGNESIUM ION) THAT REACTS WITH CALCIUM ION AND PRODUCES SALT WITH LOW SOLUBILITY

S14

CORRECTED SUPERSATURATION COEFFICIENT $S_2$ EQUAL TO OR GREATER THAN THRESHOLD VALUE $S_0$?

YES

NO

S15

NEED COUNTERMEASURE AGAINST SCALES

S16

NO NEED COUNTERMEASURE AGAINST SCALES

END

31

# FIG. 3

```
( METHOD FOR CHECKING WATER QUALITY )
```

S21

MEASURE CALCIUM ION CONCENTRATION $[Ca^{2+}]$, CARBONATE ION CONCENTRATION $[CO_3{}^{2-}]$, HYDROGEN ION CONCENTRATION $[H^+]$, WATER TEMPERATURE T, AND CONCENTRATION OF DISSOLVED ION THAT REACTS WITH CALCIUM ION, AND PRODUCES SALT WITH LOW SOLUBILITY

S22

CALCULATE SUPERSATURATION COEFFICIENT $S_1$
$S_1 = [Ca^{2+}] \, [CO_3{}^{2-}] \, f_D{}^2/K_{sp}$
*$f_D$: ACTIVITY COEFFICIENT OF DIVALENT ION (CHANGES DEPENDING ON ELECTRICAL CONDUCTIVITY (FUNCTION OF $H^+$))
*$K_{sp}$: SOLUBILITY PRODUCT (FUNCTION OF T)

S23

CALCULATE CORRECTED SUPERSATURATION COEFFICIENT $S_2$ BASED ON SUPERSATURATION COEFFICIENT $S_1$ USING MEASURED VALUE OF CONCENTRATION OF DISSOLVED ION (ORTHOPHOSPHORIC ION OR MAGNESIUM ION) THAT REACTS WITH CALCIUM ION AND PRODUCES SALT WITH LOW SOLUBILITY

S24

CALCULATE INDUCTION TIME PERIOD $t'_{ind}$ AND GROWTH SPEED $U_2$ USING CORRECTED SUPERSATURATION COEFFICIENT $S_2$, AND BASED ON VALUE OF INDUCTION TIME PERIOD $t_{ind}$, AND VALUE OF GROWTH SPEED $U_1$ THROUGH THE DEVICE IN PREDETERMINED WATER QUALITY NOT CONTAINING ORTHOPHOSPHORIC ION AND MAGNESIUM ION, AND, FURTHER CALCULATE SCALE BUILD-UP AMOUNT $V_2$ AT AN ARBITRARY USE TIME
*$t_{ind}$: TIME UNTIL SCALES ARE DEPOSITED

S25

SCALE BUILD-UP AMOUNT $V_2$ EQUAL TO OR GREATER THAN ALLOWABLE SCALE BUILDUP AMOUNT $V_0$?

YES

NO

S26

NEED COUNTERMEASURE AGAINST SCALES

S27

NO NEED COUNTERMEASURE AGAINST SCALES

( END )

# FIG. 4

METHOD FOR CHECKING WATER QUALITY

S31

MEASURE CALCIUM ION CONCENTRATION $[Ca^{2+}]$, CARBONATE ION CONCENTRATION $[CO_3^{2-}]$, HYDROGEN ION CONCENTRATION $[H^+]$, WATER TEMPERATURE T, AND CONCENTRATION OF DISSOLVED ION THAT REACTS WITH CALCIUM ION, AND PRODUCES SALT WITH LOW SOLUBILITY

S32

CALCULATE SUPERSATURATION COEFFICIENT $S_1$
$S_1 = [Ca^{2+}] [CO_3^{2-}] f_D^2/K_{sp}$
*$f_D$: ACTIVITY COEFFICIENT OF DIVALENT ION (CHANGES DEPENDING ON ELECTRICAL CONDUCTIVITY (FUNCTION OF $H^+$))
*$K_{sp}$: SOLUBILITY PRODUCT (FUNCTION OF T)

S33

CALCULATE CORRECTED SUPERSATURATION COEFFICIENT $S_2$ BASED ON SUPERSATURATION COEFFICIENT $S_1$ USING MEASURED VALUE OF CONCENTRATION OF DISSOLVED ION (ORTHOPHOSPHORIC ION OR MAGNESIUM ION) THAT REACTS WITH CALCIUM ION AND PRODUCES SALT WITH LOW SOLUBILITY

S34

CALCULATE INDUCTION TIME PERIOD $t'_{ind}$ AND GROWTH SPEED $U_2$ USING CORRECTED SUPERSATURATION COEFFICIENT $S_2$, AND BASED ON VALUE OF INDUCTION TIME PERIOD $t_{ind}$, AND VALUE OF GROWTH SPEED $U_1$ THROUGH THE DEVICE IN PREDETERMINED WATER QUALITY NOT CONTAINING ORTHOPHOSPHORIC ION AND MAGNESIUM ION
*$t_{ind}$: TIME UNTIL SCALES ARE DEPOSITED

S35

CALCULATE TIME $t'_0$ AT WHICH SCALE BUILD-UP AMOUNT REACHES ALLOWABLE SCALE BUILD-UP AMOUNT $V'_0$ BASED ON CALCULATED INDUCTION TIME PERIOD $t'_{ind}$ AND GROWTH SPEED $U_2$ (DETERMINE WATER QUALITY BASED ON TIME $t'_0$ AS INDICATOR)

END

## FIG. 5

# FIG. 6

# FIG. 7

# FIG. 8

# FIG. 9

# FIG. 10

# FIG. 11

FIBER DIAMETER

$D_2$

CURL
DIAMETER $D_1$

# FIG. 12

# FIG. 13

| | WATER TEMPE-RATURE [°C] | pH | $Na^+$ [mg/L] | $Mg^{2+}$ [mg/L] | $Cl^-$ [mg/L] | $NO_3^-$ [mg/L] | $HPO_4^{2-}$ [mg/L] | $SO_4^{2-}$ [mg/L] | Ca HARD-NESS [mg/L] | M-alkalinity [mg/L] |
|---|---|---|---|---|---|---|---|---|---|---|
| TAP WATER | 12~13.5 | 7.3~7.8 | 10.5 | 3.2 | 23.8 | 33.2 | 3.2 | 22.8 | 290~292 | 215~225 |
| SIMULATED WATER | 12~14 | 7.5~7.6 | 8.3 | 2.6 | 28.0 | 43.0 | 0.0 | 26.5 | 292 | 215~225 |
| FIRST TEST WATER ( SIMULATED WATER +$K_3PO_4$ 1mg/L) | 12~14 | 7.6~7.7 | 8.3 | 2.6 | 28.0 | 43.0 | 0.4 | 26.5 | 292 | 220~230 |
| SECOND TEST WATER ( SIMULATED WATER +$K_3PO_4$ 5mg/L) | 12~14 | 7.6~7.7 | 8.3 | 2.6 | 28.0 | 43.0 | 2.2 | 26.5 | 292 | 220~230 |

# FIG. 14

| CONDITIONS | TEMPE-RATURE (°C) | pH (−) | Ca HARDNESS ($mgCaCO_3/L$) | M-alkalinity ($mgCaCO_3/L$) |
|---|---|---|---|---|
| TAP WATER, 147 hr LATER | 65.0 | 7.9 | 260 | 180 |
| SIMULATED WATER, 146 hr LATER | 65.0 | 8.1 | 121 | 32.5 |
| FIRST TEST WATER ( SIMULATED WATER +$K_3PO_4$ 1mg/L, 263hr LATER) | 65.0 | 7.6 | 205 | 80 |
| SECOND TEST WATER ( SIMULATED WATER +$K_3PO_4$ 5mg/L, 146hr LATER) | 65.0 | 8.2 | 253 | 175 |

# FIG. 15

# FIG. 16

CORRECTED
SUPERSATURATION
COEFFICIENT $S_2$

ORTHOPHOSPHORIC ACID CONCENTRATION $C_P$[mg/L]

# FIG. 17

|  | WATER TEMPE-RATURE [°C] | pH | $Na^+$ [mg/L] | $Mg^{2+}$ [mg/L] | $Cl^-$ [mg/L] | $NO_3^-$ [mg/L] | $HPO_4^{2-}$ [mg/L] | $SO_4^{2-}$ [mg/L] | Ca HARD-NESS [mg/L] |
|---|---|---|---|---|---|---|---|---|---|
| SIMULATED WATER | 13 | 7.5~7.6 | 8.3 | 2.6 | 28.0 | 43.0 | 0.0 | 26.5 | 292 |
| THIRD TEST WATER ($^{SIMULATED}_{WATER}$ + $Mg(OH)_2$ 87mg/L) | 13 | 7.6~7.7 | 8.3 | 35.6 | 28.0 | 43.0 | 0.0 | 26.5 | 292 |
| FOURTH TEST WATER ($^{SIMULATED}_{WATER}$ + $Mg(OH)_2$ 175mg/L) | 13 | 7.6~7.7 | 8.3 | 71.3 | 28.0 | 43.0 | 0.0 | 26.5 | 292 |

## FIG. 18

**EP 2 980 584 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S61220793 A
- WO 2011045878 A
- JP 2002086190 A

**Non-patent literature cited in the description**

- **YOSHIHIKO GOTOU.** *Crystal Growth,* 29-44
- **PLUMMER et al.** *Geochimica Et Cosmochimica Acta,* 1982, vol. 46 (6), 1011-1040
- **WIECHER et al.** *Water Research,* 1975, vol. 9 (9), 835-845